# EUROPEAN PATENT APPLICATION

(11) **EP 4 360 609 A1**
(43) Date of publication of application: **01.05.2024**
(21) Application number: 22828517.7
(22) Date of filing: 23.06.2022
(51) Int. Cl.: A61K 6/60, A61K 6/15, A61K 6/30, A61K 6/71, A61K 6/831, A61K 6/887

(54) **DENTAL ADHESIVE KIT**

(30) Priority: 23.06.2021 JP 2021104488
(71) Applicant: Kuraray Noritake Dental Inc., Kurashiki-shi, Okayama 710-0801 (JP)
(72) Inventor: MATSUURA, Ryo, Tainai-shi, Niigata 959-2653 (JP); NOJIRI, Yamato, Tainai-shi, Niigata 959-2653 (JP)
(74) Representative: D Young & Co LLP
(86) International application number: PCT/JP2022/025189
(87) International publication number: WO 2022/270601

(57) **Abstract**

The present invention provides a dental adhesive kit whereby a separate-pack type dental adhesive composition exhibits excellent bond durability with respect to the surface of a one-pack type self-adhesive dental composite resin coating a prepared tooth surface. The present invention relates to a dental adhesive kit comprising: a one-pack type self-adhesive dental composite resin (X) that comprises a monomer (a-1) having a divalent phosphoric acid group with a backbone C8 to C16 alkyl or alkylene group within the molecule, a monomer (b) having no acidic group, a photopolymerization initiator (c), and a filler (d); and a separate-pack type dental adhesive composition (Y) that comprises a first agent and a second agent that are separately packed from each other, with the first agent comprising a monomer (a) having an acidic group, and a chemical polymerization initiator (e), and the second agent comprising a monomer (b) having no acidic group, wherein a cured product of the self-adhesive dental composite resin (X) after light curing has a flexural modulus ranging from 1.5 to 6 GPa, and a cured product of the separate-pack type dental adhesive composition (Y) after chemical curing has a flexural modulus ranging from 1.5 to 6 GPa.

## Description

### TECHNICAL FIELD

The present invention relates to a dental adhesive kit comprising a one-pack type self-adhesive dental composite resin and a separate-pack type dental adhesive composition. More specifically, the invention relates to a dental adhesive kit whereby a separate-pack type dental adhesive composition exhibits excellent bond durability with respect to the surface of a one-pack type self-adhesive dental composite resin coating a prepared tooth surface.

### BACKGROUND ART

Adhesive materials find application in the restorative treatment of hard body tissues in moist bodies (for example, tooth structure, bones). Resin-based curable compositions containing components such as radical polymerizable monomers and polymerization initiators are frequently used as adhesive materials in such moist body applications.

Teeth that have lost their function due to issues such as decay or accidents are restored by, for example, fixing a crown restoration material, either metal or ceramic, known as an inlay or crown, to the affected teeth. An adhesive called dental resin cement is used to fix the crown restoration material to the teeth.

In dental treatment, two restorative approaches exist: the direct restoration method involving the direct placement of a composite resin dental restoration material into the cavity, and the indirect restoration method whereby a dental prosthesis such as a metallic or ceramic crown restoration material is fixed to the tooth using a dental resin cement. In the indirect restoration method, a temporary restoration is typically placed to protect the prepared tooth structure while the crown restoration is being prepared, using a temporary sealant or a temporary bonding agent.

Given such situations, a technique has been proposed that is intended to enhance the adhesive properties of dental resin cement, improve the internal fit of the restoration, and protect the exposed dentin. In this technique, a dental adhesive and dental composite resin are applied to the prepared cavity surface immediately after the cavity is formed and before taking an impression, in order to protect the exposed dentin and tooth pulp, and a temporary restoration such as a temporary sealant or temporary bonding agent is placed to subsequently enhance the bonding of the dental resin cement to dentin (hereinafter, "resin coating method").

In recent years, there has been development of a self-adhesive dental composite resin, a type of dental composite resin that has incorporated adhesive properties. It has been introduced into practical use as a material that reduces the number of procedural steps in restorative treatments by eliminating the need for dental adhesive. Such a self-adhesive dental composite resin contains a polymerizable monomer having an acidic group, traditionally used in dental adhesives, to provide adhesive properties to tooth structure, in addition to incorporating components from conventional dental composite resin, including polyfunctional polymerizable monomers and fillers to provide mechanical strength, and polymerization initiators to improve curability (Patent Literatures 1 to 5). Non Patent Literature 1 proposes a method that uses a self-adhesive dental composite resin as a lining material for the direct restoration method.

### CITATION LIST

### Patent Literature

Patent Literature 1: JP 2008-260752 A
Patent Literature 2: JP 2017-105716 A
Patent Literature 3: JP 2018-065831 A
Patent Literature 4: US Patent Application Publication Number 2010/041786
Patent Literature 5: US Patent Application Publication Number 2011/217677 Non Patent Literature
Non Patent Literature 1: I. Baltacioglu, et al, Journal of Adhesion Science and Technology Vol.31, Issue 24 (2017) Pages 2719-2729

### SUMMARY OF INVENTION

### Technical Problem

The self-adhesive dental composite resins described in Patent Literatures 1 to 5 could be potentially utilized as a substitute for the dental adhesive and dental composite resin of the resin coating method. However, investigations by the present inventors have revealed that, while these self-adhesive dental composite resins all excel in adhesive properties to tooth structure, there is room for improvement in the bond durability of a dental resin cement to the coating surface created by the cured self-adhesive dental composite resin. Moreover, although the self-adhesive dental composite resin described in Non Patent Literature 1 demonstrates excellence as a lining material for Class II cavities, it has been identified that this self-adhesive dental composite resin has issues in the durability of its bond with dental resin cement when used with dental resin cement in the resin coating method.

It is an object of the present invention to provide a dental adhesive kit whereby a separate-pack type dental adhesive composition exhibits excellent bond durability with respect to the surface of a one-pack type self-adhesive dental composite resin coating a prepared tooth surface.

Another object of the present invention is to provide a dental adhesive kit comprising a one-pack type self-adhesive dental composite resin having excellent water resistance.

### Solution to Problem

The present inventors conducted intensive studies, and found that the foregoing issues can be overcome with a dental adhesive kit comprising a one-pack type self-adhesive dental composite resin of a specific composition, and a separate-pack type dental adhesive composition of a specific composition. The invention was completed after further studies.

Specifically, the present invention includes the following.
[1] A dental adhesive kit comprising:
   a one-pack type self-adhesive dental composite resin (X) that comprises a monomer (a-1) having a divalent phosphoric acid group with a backbone C8 to C16 alkyl or alkylene group within the molecule, a monomer (b) having no acidic group, a photopolymerization initiator (c), and a filler (d); and
   a separate-pack type dental adhesive composition (Y) that comprises a first agent and a second agent that are separately packed from each other, with the first agent comprising a monomer (a) having an acidic group, and a chemical polymerization initiator (e), and the second agent comprising a monomer (b) having no acidic group,
   wherein a cured product of the self-adhesive dental composite resin (X) after light curing has a flexural modulus ranging from 1.5 to 6 GPa, and
   a cured product of the separate-pack type dental adhesive composition (Y) after chemical curing has a flexural modulus ranging from 1.5 to 6 GPa.
[2] The dental adhesive kit according to [1], wherein a cured product of the separate-pack type dental adhesive composition (Y) after chemical curing has a flexural modulus ranging from 3 to 6 GPa.
[3] The dental adhesive kit according to [1] or [2], wherein the self-adhesive dental composite resin (X) and the separate-pack type dental adhesive composition (Y) have a flexural modulus ratio ((X)/(Y)) of 0.5 to 3.0 as a ratio of a flexural modulus of a cured product of the self-adhesive dental composite resin (X) and a flexural modulus of a cured product of the separate-pack type dental adhesive composition (Y).
[4] The dental adhesive kit according to any one of [1] to [3], wherein the content of the filler (d) is 50 mass% or more in total 100 mass% of the self-adhesive dental composite resin (X).
[5] The dental adhesive kit according to any one of [1] to [4], wherein a cured product of the self-adhesive dental composite resin (X) has a water sorption of 50 µg/mm³ or less.
[6] The dental adhesive kit according to any one of [1] to [5], wherein the monomer (a) having an acidic group contained in the separate-pack type dental adhesive composition (Y) comprises at least one selected from the group consisting of a monomer having a phosphoric acid group, a monomer having a carboxylic acid group, and a monomer having a sulfonic acid group.
[7] The dental adhesive kit according to [6], wherein the monomer (a) having an acidic group contained in the separate-pack type dental adhesive composition (Y) comprises a compound identical to the monomer (a-1) having a phosphoric acid group contained in the self-adhesive dental composite resin (X).
[8] The dental adhesive kit according to any one of [1] to [7], wherein the chemical polymerization initiator (e) comprises an organic peroxide and/or an inorganic peroxide.
[9] The dental adhesive kit according to any one of [1] to [8], wherein the first agent of the separate-pack type dental adhesive composition (Y) additionally comprises a filler (d).
[10] The dental adhesive kit according to any one of [1] to [9], wherein the first agent of the separate-pack type dental adhesive composition (Y) additionally comprises a monomer (b) having no acidic group.
[11] The dental adhesive kit according to any one of [1] to [10], wherein the first or second agent of the separate-pack type dental adhesive composition (Y) additionally comprises a silane coupling agent (g).
[12] A tooth restoration method comprising the steps of:
   curing a one-pack type self-adhesive dental composite resin (X);
   applying a separate-pack type dental adhesive composition (Y) to a predetermined region of the cured self-adhesive dental composite resin (X); and
   curing the applied separate-pack type dental adhesive composition (Y),
   the self-adhesive dental composite resin (X) comprising a monomer (a-1) having a divalent phosphoric acid group with a backbone C8 to C16 alkyl or alkylene group within the molecule, a monomer (b) having no acidic group, a photopolymerization initiator (c), and a filler (d),
   the separate-pack type dental adhesive composition (Y) comprising a first agent and a second agent that are separately packed from each other, with the first agent comprising a monomer (a) having an acidic group, and a chemical polymerization initiator (e), and the second agent comprising a monomer (b) having no acidic group.

### Advantageous Effects of Invention

According to the present invention, a dental adhesive kit can be provided whereby a separate-pack type dental adhesive composition exhibits excellent bond durability with respect to the surface of a one-pack type self-adhesive dental composite resin coating a prepared tooth surface.

According to the present invention, a dental adhesive kit can be provided that comprises a one-pack type self-adhesive dental composite resin having excellent water resistance.

According to the present invention, a dental adhesive kit can be provided that can be suitably used for indirect restoration method by virtue of the excellent bond durability of the separate-pack type dental adhesive composition with respect to the coating surface created by the cured self-adhesive dental composite resin.

### DESCRIPTION OF EMBODIMENTS

The following describes preferred embodiments of a dental adhesive kit according to the present invention. It is to be noted that the present invention is in no way limited by the descriptions of the following embodiments.

A dental adhesive kit according to the present embodiment comprises a one-pack type self-adhesive dental composite resin (X) (hereinafter, also referred to simply as "self-adhesive dental composite resin (X)") and a separate-pack type dental adhesive composition (Y), wherein a cured product of the self-adhesive dental composite resin (X) after light curing has a flexural modulus ranging from 1.5 to 6 GPa, and a cured product of the separate-pack type dental adhesive composition (Y) after chemical curing has a flexural modulus ranging from 1.5 to 6 GPa.

In this specification, the term "(meth)acryl" is a collective term for methacryl and acryl, and the same applies to similar expressions, such as "(meth)acrylic acid" and "(meth)acrylonitrile". In the present specification, the upper limits and lower limits of numeric ranges (for example, ranges of contents of components, ranges of values calculated from components, and ranges of physical properties) can be appropriately combined. Additionally, numeric ranges can be appropriately varied based on the descriptions provided in this specification. For example, concerning the content of the monomer (b) having no acidic group in the self-adhesive dental composite resin (X), the preferred range of 50 to 99 parts by mass in total 100 parts by mass of monomers in the self-adhesive dental composite resin (X) can be set to, for example, 60 to 99 parts by mass, depending on the relationship with other components, and such variations are also covered by the present invention.

In view of its adhesive properties to the separate-pack type dental adhesive composition (Y), a one-pack type self-adhesive dental composite resin (X) of the present invention has a flexural modulus of 1.5 to 6 GPa, preferably 2 to 6 GPa, more preferably 2.5 to 6 GPa, even more preferably 3 to 6 GPa in its cured product after light curing. The method of measurement of the flexural modulus of the cured product is as described in the EXAMPLES section below. The cured product of self-adhesive dental composite resin (X) cannot exhibit sufficient mechanical strength when its flexural modulus is less than 1.5 GPa. When the flexural modulus of the cured product of self-adhesive dental composite resin (X) is more than 6 GPa, detachment tends to occur at the interface between the self-adhesive dental composite resin (X) and tooth structure.

In view of its adhesive properties to the self-adhesive dental composite resin (X), a separate-pack type dental adhesive composition (Y) of the present invention has a flexural modulus of 1.5 to 6 GPa, preferably 2 to 6 GPa, more preferably 2.5 to 6 GPa, even more preferably 3 to 6 GPa in its cured product after chemical curing. The method of measurement of the flexural modulus of the cured product is as described in the EXAMPLES section below. The cured product of separate-pack type dental adhesive composition (Y) cannot exhibit sufficient mechanical strength when its flexural modulus is less than 1.5 GPa. When the flexural modulus of the cured product of separate-pack type dental adhesive composition (Y) is more than 6 GPa, exceeding the upper limit of 6 GPa for the flexural modulus of the cured product of self-adhesive dental composite resin (X), it poses a difficulty in bringing the flexural modulus of the cured product of self-adhesive dental composite resin (X) and that of the cured product of separate-pack type dental adhesive composition (Y) closer together. This may result in an increased likelihood of reduced bond durability for the separate-pack type dental adhesive composition (Y) with respect to the coating surface of self-adhesive dental composite resin (X).

The flexural modulus ratio ((X)/(Y)) between the flexural modulus of the cured product of one-pack type self-adhesive dental composite resin (X) and that of the cured product of separate-pack type dental adhesive composition (Y) ranges from 0.25 to 4.0, preferably 0.5 to 3.0, more preferably 0.7 to 2.8, even more preferably 0.85 to 2.75. Confining the flexural modulus ratio of the cured products within these ranges, and providing closer flexural modulus values for the cured product of self-adhesive dental composite resin (X) and the cured product of separate-pack type dental adhesive composition (Y) represent one of the contributing factors that enables a greater reduction of water sorption in the cured product of self-adhesive dental composite resin (X), and an improvement of bond durability for the separate-pack type dental adhesive composition with respect to the coating surface of self-adhesive dental composite resin (X).

In view of reducing a decrease in flexural modulus and in the bond durability of the separate-pack type dental adhesive composition due to sorption of water by the coating surface over the prepared tooth surface (e.g., prepared cavity surface), a self-adhesive dental composite resin (X) of the present invention has a water sorption of preferably 50 µg/mm³ or less, more preferably 45 µg/mm³ or less, even more preferably 40 µg/mm³ or less in its cured product. The method of measurement of the water sorption of the cured product is as described in the EXAMPLES section below.

In a dental adhesive kit of the present invention, the reason why the separate-pack type dental adhesive composition exhibits excellent bond durability with respect to the surface of self-adhesive dental composite resin (X) coating the prepared tooth surface remains somewhat unclear. However, the following speculation has been made.

In the bonding between the self-adhesive dental composite resin (X) and separate-pack type dental adhesive composition (Y) of the present invention, the both materials have similar flexural moduli. This is believed to alleviate stress concentration at the bonding interface, contributing to enhanced bond durability.

Deterioration due to water sorption in the cured product of self-adhesive dental composite resin (X), acting as an adherend, is another factor that has a large impact on bond durability. It is speculated that a self-adhesive dental composite resin (X) of the present invention, by comprising a monomer (a-1) having a divalent phosphoric acid group with a backbone C8 to C16 alkyl or alkylene group within the molecule, enhances bond durability by exhibiting excellent water resistance in its cured product when combined with other components.

The following describes each component used in a dental adhesive kit of the present invention.

### · One-Pack Type Self-Adhesive Dental Composite Resin (X)

A composition of a self-adhesive dental composite resin (X) of the present invention comprises a monomer (a-1) having a divalent phosphoric acid group with a backbone C8 to C16 alkyl or alkylene group within the molecule, a monomer (b) having no acidic group, a photopolymerization initiator (c), and a filler (d).

### <Monomer (a-1) Having Divalent Phosphoric Acid Group with Backbone C8 to C16 Alkyl or alkylene Group within the Molecule>

The monomer (a-1) having a divalent phosphoric acid group with a backbone C8 to C16 alkyl or alkylene group within the molecule (hereinafter, referred to as "monomer (a-1) having a phosphoric acid group") used in the present invention may be used alone, or two or more thereof may be used in appropriate combinations. A self-adhesive dental composite resin (X) of the present invention, by comprising the monomer (a-1) having a phosphoric acid group, can enhance bond durability by exhibiting superior water resistance in its cured product when combined with other components, owning to the structure of the long-chain spacer present in the backbone of the monomer (a-1) having a phosphoric acid group, in addition to the presence of a phosphoric acid group. The monomer (a-1) having a phosphoric acid group has preferably 8 to 12 carbon atoms. Specific examples of the monomer (a-1) having a phosphoric acid group are as follows.

Examples of the monomer (a-1) having a phosphoric acid group include 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and acid chlorides, alkali metal salts, and amine salts of these.

In view of achieving good bond strength to the tooth structure and good water resistance in the cured product, more preferred among the foregoing examples of monomer (a-1) having a phosphoric acid group are 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, and 12-(meth)acryloyloxydodecyl dihydrogen phosphate, even more preferably 8-(meth)acryloyloxyoctyl dihydrogen phosphate, 9-(meth)acryloyloxynonyl dihydrogen phosphate, and 10-(meth)acryloyloxydecyl dihydrogen phosphate. In view of a balance with curability, 10-(meth)acryloyloxydecyl dihydrogen phosphate is most preferred.

In view of achieving good bond strength to the tooth structure and good water resistance in the cured product, the content of the monomer (a-1) having a phosphoric acid group is preferably 1 to 40 parts by mass, more preferably 2.5 to 35 parts by mass, even more preferably 5 to 30 parts by mass in total 100 parts by mass of monomers in the self-adhesive dental composite resin (X).

### <Monomer (b) Having no Acidic Group>

Examples of the monomer (b) having no acidic group include asymmetrical acrylamide-methacrylic acid ester compounds (b-1); hydrophobic monomers (b-2) having no acidic group and having a solubility of less than 10 g/L in 25°C water (hereinafter, also referred to simply as "hydrophobic monomers (b-2)"); and hydrophilic monomers (b-3) having no acidic group and having a solubility of 10 g/L or more in 25°C water (hereinafter, also referred to simply as "hydrophilic monomers (b-3)"). The monomer (b) having no acidic group may be used alone, or two or more thereof may be used in combination. In this specification, compounds having no acidic group and containing an acrylamide group and a methacryloyloxy group are classified as asymmetrical acrylamide-methacrylic acid ester compounds (b-1), whereas compounds having no acidic group and excluded from asymmetrical acrylamide methacrylic acid ester compounds (b-1) are classified as hydrophobic monomers (b-2) or hydrophilic monomers (b-3), depending on the degree of hydrophilicity.

### · Asymmetrical Acrylamide·Methacrylic Acid Ester Compound (b-1)

A certain preferred embodiment is, for example, a self-adhesive dental composite resin (X) that additionally comprises an asymmetrical acrylamide-methacrylic acid ester compound (b-1). In view of improving properties such as the adhesive properties of the self-adhesive dental composite resin (X) to tooth structure, and allowing for easy adjustments of the mechanical strength of the cured product within the desired range, the asymmetrical acrylamide methacrylic acid ester compound (b-1) is preferably a compound represented by the following general formula (1).

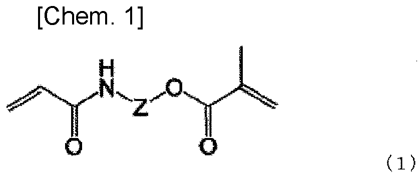

In the formula, Z is an optionally substituted linear, branched, or cyclic aliphatic group or an optionally substituted aromatic group, and the aliphatic group may be interrupted with at least one binding group selected from the group consisting of -O-, -S-, - CO-, -CO-O-, -O-CO-, -NR¹-, -CO-NR'-, -NR'-CO-, -CO-O-NR¹-, -O-CO-NR¹-, and -NR¹-CO-NR¹-. R¹ represents a hydrogen atom, or an optionally substituted linear or branched aliphatic group.

Z is a moiety that adjusts the hydrophilicity of the asymmetrical acrylamide methacrylic acid ester compound (b-1). The optionally substituted C₁ to C₈ aliphatic group represented by Z may be either a saturated aliphatic group (an alkylene group, a cycloalkylene group (for example, such as a 1 ,4-cyclohexylene group)), or an unsaturated aliphatic group (an alkenylene group, an alkynylene group). In view of availability or ease of production, and chemical stability, preferred are saturated aliphatic groups (alkylene groups). In view of adhesive properties to tooth structure and polymerization curability, Z is preferably a C₁ to C₈ aliphatic group, more preferably a C₁ to C₄ aliphatic group, even more preferably a C₂ to C₄ aliphatic group. The aliphatic group is preferably an alkylene group. Examples of the C₁ to C₈ alkylene group include a methylene group, an ethylene group, an n-propylene group, an isopropylene group, and an n-butylene group.

Examples of the optionally substituted aromatic group represented by Z include an arylene group, and an aromatic heterocyclic group. The aromatic group is preferably an arylene group rather than an aromatic heterocyclic group. The hetero ring in the aromatic heterocyclic group is normally unsaturated. The aromatic hetero ring is preferably a five-membered ring or a six-membered ring. Preferably, the arylene group is, for example, a phenylene group. Examples of the hetero ring in the aromatic heterocyclic group include a furan ring, a thiophene ring, a pyrrole ring, an oxazole ring, an isoxazole ring, a thiazole ring, an isothiazole ring, an imidazole ring, a pyrazole ring, a furazan ring, a triazole ring, a pyran ring, a pyridine ring, a pyridazine ring, a pyrimidine ring, a pyrazine ring, and a 1 ,3,5-triazine ring. The aromatic group is particularly preferably a phenylene group.

The aliphatic group represented by R¹ may be either a saturated aliphatic group (an alkyl group) or an unsaturated aliphatic group (an alkenyl group, an alkynyl group). In view of availability or ease of production, and chemical stability, preferred are saturated aliphatic groups (alkyl groups). Examples of the linear or branched alkyl group represented by R¹ include methyl, ethyl, n-propyl, isopropyl, n-butyl, isobutyl, sec-butyl, tert-butyl, n-pentyl, isopentyl, sec-pentyl, neopentyl, tert-pentyl, 1-ethylpropyl, hexyl, isohexyl, 1,1-dimethylbutyl, 2,2-dimethylbutyl, 3,3-dimethylbutyl, and 2-ethylbutyl. Preferred examples include methyl, ethyl, n-propyl, isopropyl, n-butyl, isobutyl, sec-butyl, and tert-butyl.

R¹ is preferably a hydrogen atom, or an optionally substituted linear or branched C₁ to C₈ aliphatic group, more preferably a hydrogen atom, or an optionally substituted linear or branched C₁ to C₄ alkyl group, even more preferably a hydrogen atom, or an optionally substituted linear or branched C₁ to C₃ alkyl group.

When the aliphatic group represented by Z is interrupted by the above binding group, the number of binding groups is not particularly limited, and may be about 1 to 10, preferably 1, 2, or 3, more preferably 1 or 2. In the formula (1), the aliphatic group represented by Z is preferably one that is not contiguously interrupted by the binding group. That is, the aliphatic group represented by Z is preferably one in which the binding groups are not adjacent to each other. The binding group is more preferably at least one selected from the group consisting of -O-, -S-, -CO-, -CO-O-, -O-CO-, -NH-, - CO-NH-, -NH-CO-, -CO-O-NH-, -O-CO-NH-, and -NH-CO-NH-, particularly preferably at least one selected from the group consisting of -O-, -S-, -CO-, -NH-, -CO-NH-, and -NH-CO-.

Examples of the substituents in Z include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group.

Specific examples of the asymmetric acrylamide-methacrylic acid ester compound (b-1) include, but are not particularly limited to, the following.

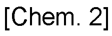

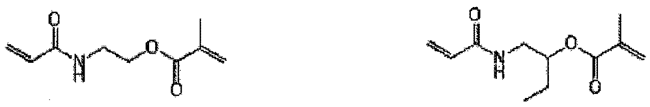

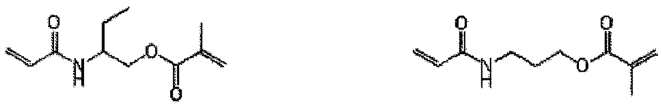

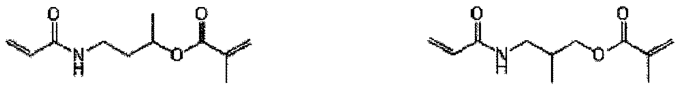

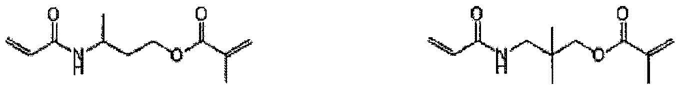

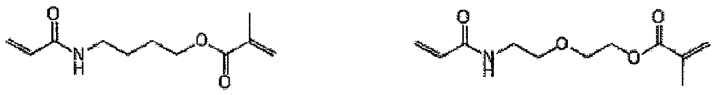

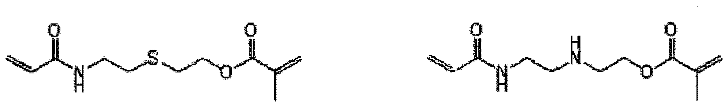

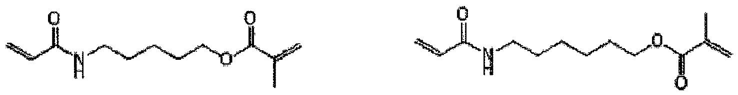

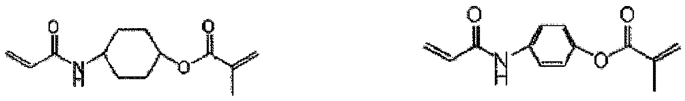

In view of adhesive properties to tooth structure and polymerization curability, preferred are asymmetric acrylamide-methacrylic acid ester compounds in which Z is an optionally substituted C₂ to C₄ linear or branched aliphatic group, more preferably N-methacryloyloxyethylacrylamide (commonly known as MAEA), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide. In view of the high hydrophilicity concerning penetration into the collagen layer of dentin, even more preferred are MAEA, and N-methacryloyloxypropylacrylamide.

The asymmetric acrylamide-methacrylic acid ester compound (b-1) may be contained alone, or two or more thereof may be contained in combination. The content of the asymmetric acrylamide-methacrylic acid ester compound (b-1) in the self-adhesive dental composite resin (X) is not particularly limited, as long as the present invention can exhibit its effects. However, the content of asymmetric acrylamide-methacrylic acid ester compound (b-1) is preferably 1 to 60 parts by mass, more preferably 2 to 45 parts by mass, even more preferably 3 to 30 parts by mass, particularly preferably 5 to 25 parts by mass in total 100 parts by mass of monomers in a self-adhesive dental composite resin (X) of the present invention.

### . Hydrophobic Monomer (b-2) Having no Acidic Group

The hydrophobic monomer (b-2) having no acidic group improves properties such as ease of handling of the self-adhesive dental composite resin (X), and the mechanical strength of the cured product. The hydrophobic monomer (b-2) is preferably a radical monomer having no acidic group but having a polymerizable group. In view of ease of radical polymerization, preferred as the polymerizable group are (meth)acryl groups and/or (meth)acrylamide groups. The hydrophobic monomer (b-2) means a monomer that has no acidic group, and does not correspond to the asymmetric acrylamide-methacrylic acid ester compound (b-1), and that has a solubility of less than 10 g/L in 25°C water. Examples of the hydrophobic monomer (b-2) include crosslinkable monomers such as aromatic bifunctional monomers, aliphatic bifunctional monomers, and tri- and higher-functional monomers.

### Examples of the aromatic bifunctional monomers include aromatic di(meth)acrylates represented by the following general formula (2).

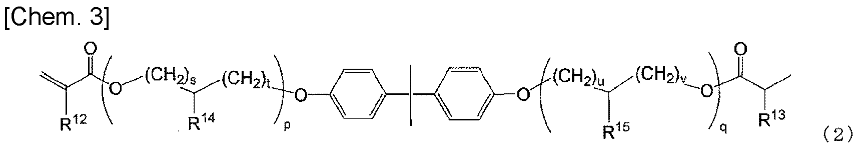

In the formula, R¹² and R¹³ are hydrogen atoms or methyl groups, R¹⁴ and R¹⁵ are each independently a hydrogen atom, a hydroxyl group, or a C1 to C3 alkyl group, s, t, u, and v are integers of 0 to 6, and p and q are integers of 0 to 8, and may be the same or different.

Specific examples of the aromatic bifunctional monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred are 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional monomers include erythritol di(meth)acrylate, sorbitol di(meth)acrylate, mannitol di(meth)acrylate, pentaerythritol di(meth)acrylate, dipentaerythritol di(meth)acrylate, polyethylene glycol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, and 2,2,4-trimethylhexamethylenebis(2-carbamoyloxyethyl)di(meth)acrylate. Preferred are glycerol di(meth)acrylate, 1 ,6-hexanediol di(meth)acrylate, triethylene glycol diacrylate, triethylene glycol dimethacrylate (commonly known as 3G), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA), and 1,10-decanediol dimethacrylate (commonly known as DD).

Examples of the tri- and higher-functional monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred is N,N-(2,2,4-trimethylhexamethylene)bis[2-(aminocarboxy)propane-1,3-diol]tetramethacrylate.

In view of mechanical strength and ease of handling, preferred among the foregoing hydrophobic monomers (b-2) are aromatic bifunctional monomers, and aliphatic bifunctional monomers. From this perspective, preferred as the aromatic bifunctional monomers are Bis-GMA and D-2.6E. From the above perspective, preferred as the aliphatic bifunctional monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, and UDMA.

In view of providing good adhesive properties to tooth structure when the hydrophobic monomer (b-2) is used as a component of the self-adhesive dental composite resin (X), the hydrophobic monomer (b-2) is more preferably Bis-GMA, D-2.6E, 3G, UDMA, or DD, even more preferably D-2.6E, 3G, or Bis-GMA.

The hydrophobic monomer (b-2) may be contained alone, or two or more thereof may be used in combination. The content of the hydrophobic monomer (b-2) in the self-adhesive dental composite resin (X) is preferably 20 to 98 parts by mass, more preferably 40 to 95 parts by mass, even more preferably 60 to 92 parts by mass in total 100 parts by mass of monomers in the self-adhesive dental composite resin (X). When the content of hydrophobic monomer (b-2) is at or below the foregoing upper limits, a decrease of adhesive properties due to reduced wettability of the self-adhesive dental composite resin (X) to tooth structure can more easily be reduced, whereas the cured product can more easily exhibit the desired mechanical strength when the content of hydrophobic monomer (b-2) is at or above the foregoing lower limits.

### · Hydrophilic Monomer (b-3) Having no Acidic Group

A self-adhesive dental composite resin (X) of the present invention preferably comprises a hydrophilic monomer (b-3) having no acidic group. The hydrophilic monomer (b-3) improves the wettability of self-adhesive dental composite resin (X) to tooth structure. The hydrophilic monomer (b-3) is preferably a radical monomer having no acidic group but having a polymerizable group. In view of ease of radical polymerization, preferred as the polymerizable group are (meth)acryl groups and/or (meth)acrylamide groups. The hydrophilic monomer (b-3) means a monomer that has no acidic group, and does not correspond to the asymmetric acrylamide-methacrylic acid ester compound (b-1), and that has a solubility of 10 g/L or more in 25°C water. The hydrophilic monomer (b-3) is preferably one having a solubility of 30 g/L or more in 25°C water, more preferably one that can dissolve in water in any proportions at 25°C. The hydrophilic monomer is preferably one having a hydrophilic group such as a hydroxyl group, an oxymethylene group, an oxyethylene group, an oxypropylene group, or an amide group. Examples of the hydrophilic monomer (b-3) include hydrophilic monofunctional (meth)acrylate monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with nine or more oxyethylene groups); and hydrophilic monofunctional (meth)-trihydroxymethyl-N-methyl(meth)acrylamide, N,N-dimethylacrylamide, and acrylamide monomers, such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, and N,N-diethylacrylamide.

In view of adhesive properties to tooth structure, preferred as hydrophilic monomer (b-3) are 2-hydroxyethyl (meth)acrylate, 2,3-dihydroxypropyl (meth)acrylate, and hydrophilic monofunctional (meth)acrylamide monomers, more preferably 2-hydroxyethyl (meth)acrylate, N,N-dimethylacrylamide, and N,N-diethylacrylamide. The hydrophilic monomer (b-3) may be contained alone, or two or more thereof may be used in combination.

The content of the hydrophilic monomer (b-3) in the self-adhesive dental composite resin (X) ranges preferably from 0 to 50 parts by mass, more preferably from 0 to 40 parts by mass, even more preferably from 0 to 30 parts by mass in total 100 parts by mass of monomers in the self-adhesive dental composite resin (X). The content of hydrophilic monomer (b-3) may be 0 part by mass in total 100 parts by mass of monomers in the self-adhesive dental composite resin (X). When the content of the hydrophilic monomer (b-3) in a self-adhesive dental composite resin (X) of the present invention is at or above these lower limits, it is easier to produce a sufficient adhesion improving effect, whereas the cured product can more easily exhibit the desired mechanical strength when the content of hydrophilic monomer (b-3) is at or below the foregoing upper limits.

The content of the monomer (b) having no acidic group in the self-adhesive dental composite resin (X) is preferably 50 to 99 parts by mass, more preferably 60 to 97.5 parts by mass, even more preferably 70 to 95 parts by mass in total 100 parts by mass of monomers in the self-adhesive dental composite resin (X). In view of adhesive properties to tooth structure, the hydrophobic monomer (b-2) and hydrophilic monomer (b-3) have a mass ratio ((b-2):(b-3)) of preferably 10:0 to 1:2, more preferably 10:0 to 1:1, even more preferably 10:0 to 2:1.

A certain preferred embodiment is, for example, a self-adhesive dental composite resin (X) that is essentially free of a bi- or higher functional (meth)acrylamide monomer. Another certain preferred embodiment is, for example, a self-adhesive dental composite resin (X) that is essentially free of a tri- or higher-functional (meth)acrylamide monomer. In this specification, "being essentially free of a polymerizable compound" means that the content of the polymerizable compound is less than 0.5 parts by mass, preferably less than 0.1 parts by mass, more preferably less than 0.01 parts by mass in total 100 parts by mass of polymerizable compounds contained in a composition of the self-adhesive dental composite resin (X) or in a composition of the separate-pack type dental adhesive composition (Y). The polymerizable compound content may be 0 part by mass. The content of the polymerizable compound that is essentially absent may be less than 0.5 mass%, or less than 0.1 mass% in the whole composition. In this specification, "consisting essentially of a certain specific component" means that components other than the specific component are essentially absent. For example, the content of components other than the specific component is preferably less than 5.0 mass%, more preferably less than 1.0 mass%, even more preferably less than 0.5 mass%, particularly preferably less than 0.1 mass%.

Another certain preferred embodiment is, for example, a self-adhesive dental composite resin (X) that is essentially free of a (meth)acrylic block copolymer. The (meth)acrylic block copolymer may have a molecular weight distribution (weight-average molecular weight/number average molecular weight) of, for example, 1.02 to 2.00. The molecular weight distribution can be measured using a known method, for example, gel permeation chromatography (GPC), and is calculated as a value equivalent to standard polystyrene. The (meth)acrylic block copolymer may be bifunctional or higher functional, or may be tetrafunctional or higher functional.

### <Photopolymerization Initiator (c)>

The photopolymerization initiator (c) can be classified into a water-soluble photopolymerization initiator (c-1) and a water-insoluble photopolymerization initiator (c-2). The photopolymerization initiator (c-2) may be solely a water-soluble photopolymerization initiator (c-1) or a water-insoluble photopolymerization initiator (c-2), or may be a combination of a water-soluble photopolymerization initiator (c-1) and a water-insoluble photopolymerization initiator (c-2). Preferably, a water-soluble photopolymerization initiator (c-1) and a water-insoluble photopolymerization initiator (c-2) are used in combination.

### · Water-Soluble Photopolymerization Initiator (c-1)

With a water-soluble photopolymerization initiator (c-1), a high bond strength can be achieved by improving polymerization curability at the interface with hydrophilic tooth surfaces. The water-soluble photopolymerization initiator (c-1) has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator (c-1) can sufficiently dissolve in water within the tooth structure at the bonding interface, and more easily exhibits the polymerization promoting effect.

Examples of the water-soluble photopolymerization initiator (c-1) include water-soluble thioxanthones; water-soluble acylphosphine oxides; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO-Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO-Na⁺ introduced into the phenyl group of 2-hydroxy-2-methyl-1-phenylpropan-1-one. Other examples of the water-soluble photopolymerization initiator (c-1) include quaternary ammonium compounds prepared by quaternization of the amino group of α-aminoalkylphenones, such as 2-methyl-1-[4-(methylthio)phenyl]-2-morpholinopropan-1-one, and 2-benzyl-2-(dimethylamino)-1-[(4-morpholino)phenyl]-1-butanon.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneaminium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propaneaminium chloride.

Examples of the water-soluble acylphosphine oxides include acylphosphine oxides represented by the following general formula (3) or (4).

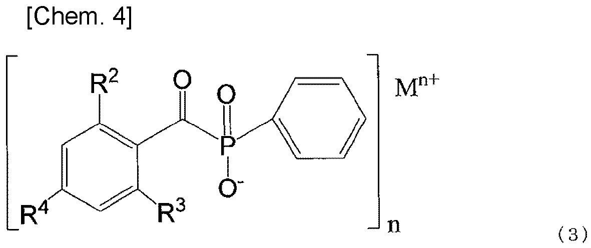

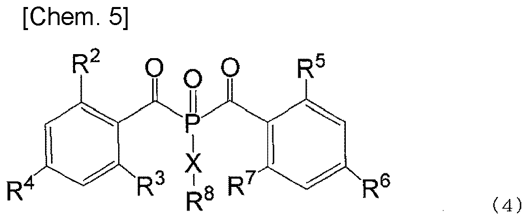

In formulae (3) and (4), R², R³, R⁴, R⁵, R⁶, and R⁷ are each independently a C₁ to C₄ linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by HN⁺R⁹R¹⁰R¹¹ (where R⁹, R¹⁰, and R¹¹ are each independently an organic group or a hydrogen atom), n is 1 or 2, X is a C₁ to C₄ linear or branched alkylene group, and R⁸ represents - CH(CH₃)COO(C₂H₄O)_{P}CH₃, where p represents an integer of 1 to 1,000.

The alkyl group represented by R², R³, R⁴, R⁵, R⁶, and R⁷ is not particularly limited, as long as it is a C₁ to C₄ linear or branched alkyl group. Examples include methyl, ethyl, n-propyl, isopropyl, n-butyl, isobutyl, sec-butyl, 2-methylpropyl, and tert-butyl. The alkyl group represented by R², R³, R⁴, R⁵, R⁶, and R⁷ is preferably a C₁ to C₃ linear alkyl group, more preferably methyl or ethyl, even more preferably methyl. Examples of X include a methylene group, an ethylene group, an n-propylene group, an isopropylene group, and an n-butylene group. X is preferably a C₁ to C₃ linear alkylene group, more preferably a methylene group or an ethylene group, even more preferably a methylene group.

Examples of the substituent of the pyridine ring when M is a pyridinium ion include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group. Preferably, M is an alkali metal ion, an alkali earth metal ion, a magnesium ion, a pyridinium ion (the pyridine ring may have a substituent), or an ammonium ion represented by HN⁺R⁹R¹⁰R¹¹ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic group represented by R⁹, R¹⁰, and R¹¹ may be the same group exemplified above for the substituent of the pyridine ring (excluding a halogen atom).

In view of storage stability and shade stability in a composition of the self-adhesive dental composite resin (X), particularly preferred are compounds in which R², R³, R⁴, R⁵, R⁶, and R⁷ are all methyl groups. Examples of ammonium ions include ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine.

In view of adhesive properties, p in R⁸ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferred among these water-soluble acylphosphine oxides are lithium phenyl(2,4,6-trimethylbenzoyl)phosphinate, sodium phenyl(2,4,6-trimethylbenzoyl)phosphinate, and compounds represented by general formula (4) and in which the moiety corresponding to the group represented by R⁸ is synthesized from polyethylene glycol methyl ether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (c-1) may be used alone, or two or more thereof may be used in combination.

The water-soluble photopolymerization initiator (c-1) may be dissolved in the self-adhesive dental composite resin (X), or may be dispersed in the form of a powder in a composition of the self-adhesive dental composite resin (X).

When the water-soluble photopolymerization initiator (c-1) is dispersed in the form of a powder, the average particle diameter is preferably 500 µm or less, more preferably 100 µm or less, even more preferably 50 µm or less because the water-soluble photopolymerization initiator (c-1) tends to settle when the average particle diameter of the powder is excessively large. The average particle diameter is preferably 0.01 µm or more because the specific surface area of the powder overly increases, and the amount of powder that can be dispersed in the composition decreases when the average particle diameter of the powder is excessively small. Taken together, the average particle diameter of water-soluble photopolymerization initiator (c-1) preferably ranges from 0.01 to 500 µm, more preferably 0.01 to 100 µm, even more preferably 0.01 to 50 µm.

The average particle diameter of a powder of individual water-soluble photopolymerization initiators (c-1) can be determined by taking an electron micrograph of at least 100 particles, and calculating the volume average particle diameter from the captured image after an image analysis with image-analyzing particle size distribution measurement software (Mac-View, manufactured by Mountech Co., Ltd.).

The particle shape of the water-soluble photopolymerization initiator (c-1) when it is dispersed in the form of a powder is not particularly limited, and may be any of various shapes, including, for example, spherical, stylus, plate-like, and crushed shapes. The water-soluble photopolymerization initiator (c-1) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the self-adhesive dental composite resin (X) obtained, the content of water-soluble photopolymerization initiator (c-1) is preferably 0.01 to 20 parts by mass relative to total 100 parts by mass of monomers in a self-adhesive dental composite resin (X) of the present invention. In view of adhesive properties to tooth structure, the content of water-soluble photopolymerization initiator (c-1) is more preferably 0.05 to 10 parts by mass, even more preferably 0.1 to 5 parts by mass relative to total 100 parts by mass of monomers in a self-adhesive dental composite resin (X) of the present invention. When the content of water-soluble photopolymerization initiator (c-1) is at or above these lower limits, polymerization can sufficiently proceed at the bonding interface, making it easier to provide a sufficient bond strength. When the content of water-soluble photopolymerization initiator (c-1) is at or below the foregoing upper limits, it is easier to provide a sufficient bond strength.

### · Water-Insoluble Photopolymerization Initiator (c-2)

In view of curability, a self-adhesive dental composite resin (X) of the present invention preferably comprises a water-insoluble photopolymerization initiator (c-2) having a solubility of less than 10 g/L in 25°C water (hereinafter, also referred to as "water-insoluble photopolymerization initiator (c-2)"), other than the water-soluble photopolymerization initiator (c-1). The water-insoluble photopolymerization initiator (c-2) used in the present invention may be a known photopolymerization initiator. The water-insoluble photopolymerization initiator (c-2) may be contained alone, or two or more thereof may be contained in combination.

Examples of the water-insoluble photopolymerization initiator (c-2) include (bis)acylphosphine oxides (other than those exemplified for the water-soluble photopolymerization initiator (c-1)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and salts of these.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Example of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these examples, the water-insoluble photopolymerization initiator (c-2) is preferably at least one selected from the group consisting of a (bis)acylphosphine oxide, an α-diketone, and a coumarin. In this way, a self-adhesive dental composite resin (X) can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (c-2) is not particularly limited. However, in view of curability and other properties of the composition of self-adhesive dental composite resin (X) obtained, the content of water-insoluble photopolymerization initiator (c-2) is preferably 0.01 to 10 parts by mass, more preferably 0.05 to 7 parts by mass, even more preferably 0.1 to 5 parts by mass relative to total 100 parts by mass of monomers in a self-adhesive dental composite resin (X) of the present invention. When the content of water-insoluble photopolymerization initiator (c-2) is at or below these upper limits, it is easier to obtain a sufficient bond strength when the water-insoluble photopolymerization initiator (c-2) itself has low polymerization performance, in addition to reducing the precipitation of the water-insoluble photopolymerization initiator (c-2) itself from the self-adhesive dental composite resin (X).

When the water-soluble photopolymerization initiator (c-1) and the water-insoluble photopolymerization initiator (c-2) are used in combination, the mass ratio (c-1):(c-2) of water-soluble photopolymerization initiator (c-1) and water-insoluble photopolymerization initiator (c-2) in the present invention is preferably 10:1 to 1:10, more preferably 7:1 to 1:7, even more preferably 5:1 to 1:5, particularly preferably 3:1 to 1:3. When the fraction of water-soluble photopolymerization initiator (c-1) in the mass ratio exceeds 10:1, the curability of the self-adhesive dental composite resin (X) itself may decrease, and the self-adhesive dental composite resin (X) may have difficulty in exhibiting high bond strength. When the fraction of water-insoluble photopolymerization initiator (c-2) in the mass ratio exceeds 1:10, the self-adhesive dental composite resin (X) may have difficulty in exhibiting high bond strength as a result of insufficient promotion of polymerization at the bonding interface, though the curability of the self-adhesive dental composite resin (X) itself can increase.

### <Filler (d)>

A self-adhesive dental composite resin (X) of the present invention must comprise a filler (d), in order to adjust the ease of handling, and to increase the mechanical strength of the cured product. Examples of the filler (d) include an inorganic filler, an organic-inorganic composite filler, and an organic filler. The filler (d) may be used alone, or two or more thereof may be used in combination.

Examples of the materials of the inorganic filler include quartz; silica; minerals containing silica as a base material, such as kaolin, clay, *ummo,* and mica; alumina; composite oxides (for example, silica-containing composite oxides such as silica-titania, silica-titania-barium oxide, silica-zirconia, silica-alumina, and silica-alumina-zirconia); various types of glass (glass containing silica as a base material, such as fused silica, lanthanum glass, borosilicate glass, soda glass, zinc glass, strontium glass, aluminosilicate glass, strontium boroaluminosilicate glass, fluoroaluminosilicate glass, calcium fluoroaluminosilicate glass, strontium fluoroaluminosilicate glass, strontium calcium fluoroaluminosilicate glass, and barium glass (such as bariumsilicate glass, barium boroaluminosilicate glass, and barium fluoroaluminosilicate glass)); ceramics (for example, ceramics containing silica as a base material); ytterbium oxide; yttrium oxide; zirconia; calcium phosphate; barium sulfate; aluminum hydroxide; and silica-coated ytterbium fluoride. These may be used alone, or two or more thereof may be used as a mixture. In view of providing superior mechanical strength and superior transparency to the self-adhesive dental composite resin (X) obtained, preferred are quartz, silica, composite oxides, glass containing silica as a base material, ceramics containing silica as a base material, ytterbium oxide, and silica-coated ytterbium fluoride, more preferably quartz, silica, silica-zirconia, barium glass, ytterbium oxide, and silica-coated ytterbium fluoride, even more preferably quartz, silica, silica-zirconia, barium glass, and silica-coated ytterbium fluoride. In view of considerations such as the ease of handling and mechanical strength of the self-adhesive dental composite resin (X) obtained, the average particle diameter of the inorganic filler is preferably 0.001 to 50 µm, more preferably 0.001 to 10 µm. In this specification, the average particle diameter of inorganic filler means the average particle diameter before surface treatment when the inorganic filler is subjected to a surface treatment, as will be described.

A certain preferred embodiment is, for example, a dental adhesive kit in which the filler (d) of the self-adhesive dental composite resin (X) comprises an inorganic filler.

The inorganic filler may be a commercially available product. Examples of such commercially available products include Aerosil^{®} OX50, Aerosil^{®} 50, Aerosil^{®} 200, Aerosil^{®} 380, Aerosil^{®} R972, and Aerosil^{®} 130, all manufactured by Nippon Aerosil Co., Ltd. under these trade names.

The shape of the inorganic filler is not particularly limited, and the particle diameter of filler may be appropriately selected for use. For example, the inorganic filler may be an irregularly shaped filler or a spherical filler. In view of improving the mechanical strength of a cured product of the self-adhesive dental composite resin (X), the inorganic filler used is preferably a spherical filler. Here, the spherical filler represents particles that appear round in shape when observed in a unit field of an electron micrograph, and that have an average uniformity of 0.6 or more as calculated by dividing the diameter of a particle perpendicular to its maximum diameter by the maximum diameter. The spherical filler has an average particle diameter of preferably 0.05 to 5 µm. When the average particle diameter is less than 0.05 µm, the mechanical strength may decrease as a result of a decrease of the filling rate of the spherical filler in the self-adhesive dental composite resin (X). When the average particle diameter is more than 5 µm, the spherical filler has a reduced surface area, and the self-adhesive dental composite resin (X) may fail to produce a cured product having high mechanical strength.

In order to adjust the flowability of the self-adhesive dental composite resin (X), the inorganic filler may be used after an optional surface treatment with a known surface treatment agent. The surface treatment agent may be, for example, a silane coupling agent (g). Examples of the silane coupling agent (g) include silane coupling agents represented by the following general formula (5).

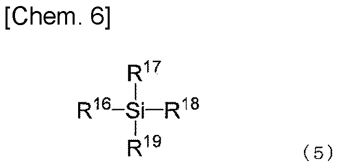

In the formula, R¹⁶ represents a C1 to C20 organic group having at least one functional group selected from the group consisting of a (meth)acryloyl group, a vinyl group, and an epoxy group; a (meth)acryloyl group; a vinyl group; or an epoxy group. The organic group is not particularly limited, and may be, for example, a saturated or unsaturated aliphatic, alicyclic, or aromatic hydrocarbon group. Particularly preferred are C3 to C15 alkylene groups. The organic group may have a substituent that does not contain a carbon atom, such as a halogen atom, a hydroxyl group, an amino group, a mercapto group, a cyano group, and a nitro group. The organic group may contain a bond other than the carbon-carbon bond within its structure, for example, such as an ether bond, an ester bond, an amide bond, a sulfonyl bond, a urethane bond, or a thioether bond. R¹⁶ may be, for example, a (meth)acryloyloxyalkyl group, a glycidyloxyalkyl group, an aminoalkyl group, or a mercaptoalkyl group. R¹⁷ represents a hydroxyl group, a halogen atom, a C1 to C5 alkyl group, or a C1 to C5 alkoxy group. The alkyl group represented by R¹⁷ may be linear, branched, or cyclic. Examples include a methyl group, an ethyl group, an n-propyl group, an isopropyl group, a cyclopropyl group, an n-butyl group, an isobutyl group, a sec-butyl group, a tert-butyl group, a cyclobutyl group, and an n-pentyl group. R¹⁸ and R¹⁹ each represent a hydroxyl group, a halogen atom, or a C1 to C5 alkoxy group. The alkoxy groups represented by R¹⁷, R¹⁸, and R¹⁹ may be linear, branched, or cyclic. Examples include a methoxy group, an ethoxy group, an n-propoxy group, an isopropoxy group, a cyclopropoxy group, an n-butoxy group, a cyclobutoxy group, a tert-butoxy group, an n-pentyl group, and a cyclopentyloxy group. Preferably, at least one of R¹⁷ to R¹⁹ is a C2 to C5 alkoxy group.

The silane coupling agent (g) is used alone, or two or more thereof are used in combination. The silane coupling agent (g) may be any known silane coupling agent, preferably a silane coupling agent represented by general formula (5). Specific examples of silane coupling agent (g) include vinyltrimethoxysilane, vinyltriethoxysilane, vinyltrichlorosilane, vinyltris(β-methoxyethoxy)silane, vinyltripropoxysilane, vinyltributoxysilane, γ-methacryloyloxypropyltrimethoxysilane, γ-methacryloyloxypropyltriethoxysilane, γ-methacyloyloxypropyltris(β-methoxyethoxy)silane, 6-(meth)acryloyloxyhexyltrimethoxysilane, 6-(meth)acryloyloxyhexyltriethoxysilane, 8-(meth)acryloyloxyoctyltrimethoxysilane, 8-(meth)acryloyloxyoctyltriethoxysilane, κ-methacryloyloxydecyltrimethoxysilane, κ-methacryloyloxydecyltriethoxysilane, 11-(meth)acryloyloxyundecyltrimethoxysilane, 11-(meth)acryloyloxyundecyltriethoxysilane, γ-aminopropyltriethoxysilane, and (γ-mercaptopropyl)trimethoxysilane.

The surface treatment can be carried out using any known method, including, for example, a method that adds the surface treatment agent by spraying it while vigorously stirring the inorganic filler, a method that disperses or dissolves the inorganic filler and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, a surface treatment can be achieved by applying heat in a temperature range of typically 50 to 150°C to allow the reaction between the surface of inorganic filler and the surface treatment agent to proceed to completion. The amount of surface treatment agent is not particularly limited, and the surface treatment agent may be used in an amount of 1 to 10 parts by mass relative to 100 parts by mass of inorganic filler before treatment.

The organic-inorganic composite filler used in the present invention is a filler obtained by adding a monomer to the inorganic filler, polymerizing the mixture in paste form, and pulverizing the polymerized filler. The organic-inorganic composite filler may be, for example, a TMPT filler (a filler obtained by mixing trimethylolpropane methacrylate and a silica filler, and pulverizing the mixture after polymerization). The shape of the organic-inorganic composite filler is not particularly limited, and the particle diameter of filler may be appropriately selected for use. In view of properties such as the ease of handling and mechanical strength of the composition obtained, the organic-inorganic composite filler has an average particle diameter of preferably 0.001 to 50 µm, more preferably 0.001 to 10 µm.

Examples of the materials of the organic filler include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of organic filler is not particularly limited, and the particle diameter of filler may be appropriately selected for use. In view of considerations such as the ease of handling and mechanical strength of the self-adhesive dental composite resin (X) obtained, the average particle diameter of the organic filler is preferably 0.001 to 50 µm, more preferably 0.001 to 10 µm.

In this specification, the average particle diameter of filler can be determined by a laser diffraction scattering method or by observing particles with an electron microscope. Specifically, a laser diffraction scattering method is more convenient for the measurement of particles having a particle size of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

In a self-adhesive dental composite resin (X) of the present invention, it is preferable to use a mixture or a combination of two or more fillers of different materials, different particle size distributions, and different forms. By combining two or more types of fillers, the fillers can interact with the monomer or with the other fillers at an increased number of points, in addition to densely packing themselves. Depending on the type of filler, it is also possible to control paste fluidity in the presence or absence of a shear force. In view of the ease of handling and paste properties of a self-adhesive dental composite resin (X) of the present invention, the filler (d) is preferably a combination (I) of a filler (d-1) having an average particle diameter of 1 nm or more and less than 0.1 µm, and a filler (d-2) having an average particle diameter of 0.1 µm or more and 1 µm or less; a combination (II) of a filler (d-1) having an average particle diameter of 1 nm or more and less than 0.1 µm, and a filler (d-3) having an average particle diameter of more than 1 µm and 10 µm or less; a combination (III) of a filler (d-1) having an average particle diameter of 1 nm or more and less than 0.1 µm, a filler (d-2) having an average particle diameter of 0.1 µm or more and 1 µm or less, and a filler (d-3) having an average particle diameter of more than 1 µm and 10 µm or less; or a combination (IV) of fillers (d-2) having an average particle diameter of 0.1 µm or more and 1 µm or less. In view of the paste properties of self-adhesive dental composite resin (X), the combinations (I), (II), and (III) are more preferred, and the combinations (I) and (II) are even more preferred. The combination (IV) of fillers (d-2) having an average particle diameter of 0.1 µm or more and 1 µm or less means an embodiment that comprises two types of fillers (d-2) of different average particle diameters between 0.1 µm and 1 µm. The fillers (d) of different particle diameters may include other types of fillers, provided that the fillers (d) have the foregoing combinations. Unintended inclusion of non-filler particles as impurities is acceptable to such an extent that it does not hinder the effectiveness of the present invention.

The content of filler (d) is not particularly limited. In view of the mechanical strength of the cured product and adhesive properties to tooth structure, the content of filler (d) is preferably 50 mass% or more, more preferably 50 to 90 mass%, even more preferably 55 to 85 mass%, particularly preferably 60 to 80 mass% in total 100 mass% of the self-adhesive dental composite resin (X).

A method of production of a self-adhesive dental composite resin (X) of the present invention is not particularly limited, as long as it produces a self-adhesive dental composite resin (X) comprising a monomer (a-1) having a divalent phosphoric acid group with a backbone C8 to C16 alkyl or alkylene group within the molecule, a monomer (b) having no acidic group, a photopolymerization initiator (c), and a filler (d). A self-adhesive dental composite resin (X) of the present invention can be produced with ease using a method known to a person skilled in the art.

### <Polymerization Accelerator(f)>

A self-adhesive dental composite resin (X) of the present invention may use a polymerization accelerator (f), along with the water-insoluble photopolymerization initiator (c-2) and/or a chemical polymerization initiator (described later). Examples of the polymerization accelerator (f) used in the self-adhesive dental composite resin (X) include amines, sulfinic acid and salts thereof, benzotriazole compounds, benzoimidazole compounds, sulfur-containing reducing inorganic compounds, thiourea compounds, aldehydes, thiol compounds, borate compounds, barbituric acid compounds, triazine compounds, copper compounds, tin compounds, vanadium compounds, and halogen compounds. Preferred are amines, sulfinic acid and salts thereof, sulfur-containing reducing inorganic compounds, thiourea compounds, aldehydes, thiol compounds, borate compounds, barbituric acid compounds, triazine compounds, copper compounds, tin compounds, vanadium compounds, and halogen compounds.

The amines used as polymerization accelerator (f) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the self-adhesive dental composite resin (X), preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart excellent curability to the self-adhesive dental composite resin (X), it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Examples of the sulfinic acid and salts thereof include benzenesulfinic acid, p-toluenesulfinic acid, o-toluenesulfinic acid, ethylbenzenesulfinic acid, decylbenzenesulfinic acid, and dodecylbenzenesulfinic acid; and lithium salts, sodium salts, potassium salts, rubidium salts, cesium salts, magnesium salts, calcium salts, strontium salts, iron salts, zinc salts, ammonium salts, tetramethylammonium salts, and tetraethylammonium salts of 2,4,6-trimethylbenzenesulfinic acid, 2,4,6-triisopropylbenzene sulfinic acid (hereinafter, sodium salts may be abbreviated as TPBSS), chlorobenzenesulfinic acid, and naphthalenesulfinic acid. In view of the curability and storage stability of the composition, preferred are lithium salts, sodium salts, potassium salts, magnesium salts, and calcium salts of 2,4,6-trimethylbenzenesulfinic acid and 2,4,6-triisopropylbenzenesulfinic acid, more preferably lithium salts, sodium salts, potassium salts, magnesium salts, and calcium salts of 2,4,6-triisopropylbenzenesulfinic acid.

Examples of the benzotriazole compounds and/or benzoimidazole compounds include compounds represented by the following general formula (6), and compounds represented by the following general formula (7), respectively.

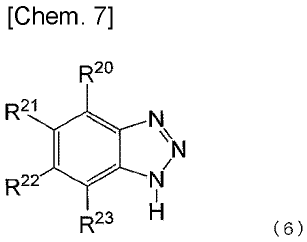

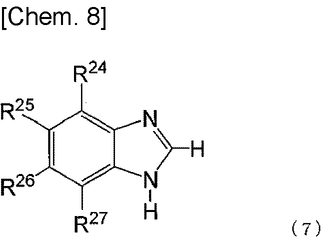

In the general formulae (6) and (7), R²⁰ to R²⁷ each independently represent a hydrogen atom, a hydroxyl group, an alkyl group, an aryl group, an alkoxy group, an alkenyl group, an aralkyl group, or a halogen atom.

The alkyl groups represented by R²⁰ to R²⁷ may be linear, branched, or cyclic, preferably with 1 to 10 carbon atoms. Specific examples include methyl, ethyl, n-propyl, isopropyl, cyclopropyl, n-butyl, isobutyl, sec-butyl, tert-butyl, cyclobutyl, n-pentyl, isopentyl, neopentyl, tert-pentyl, cyclopentyl, n-hexyl, isohexyl, cyclohexyl, n-heptyl, cycloheptanyl, n-octyl, 2-ethylhexyl, cyclooctyl, n-nonyl, cyclononyl, and n-decyl. Particularly preferred are methyl and ethyl.

The aryl groups represented by R²⁰ to R²⁷ are preferably aryl groups with 6 to 14 carbon atoms, for example, such as a phenyl group, a naphthyl group, and an anthryl group.

The alkoxy groups represented by R²⁰ to R²⁷ may be linear, branched, or cyclic, preferably with 1 to 8 carbon atoms. Specific examples include a methoxy group, an ethoxy group, an n-propoxy group, an isopropoxy group, an n-butoxy group, a tert-butoxy group, an n-hexyloxy group, a cyclohexyloxy group, an n-octyloxy group, and a 2-ethylhexyloxy group.

The alkenyl groups represented by R²⁰ to R²⁷ may be linear, branched, or cyclic, preferably with 1 to 6 carbon atoms. Specific examples include a vinyl group, an allyl group, a methylvinyl group, a propenyl group, a butenyl group, a pentenyl group, a hexenyl group, a cyclopropenyl group, a cyclobutenyl group, a cyclopentenyl group, and a cyclohexenyl group.

Examples of the aralkyl groups represented by R²⁰ to R²⁷ include alkyl groups (particularly, an alkyl group having 1 to 10 carbon atoms) substituted with an aryl group (particularly, an aryl group having 6 to 10 carbon atoms). A specific example is a benzyl group.

Examples of the halogen atoms represented by R²⁰ to R²⁷ include a fluorine atom, a chlorine atom, a bromine atom, and an iodine atom.

R²⁰ to R²⁷ are preferably hydrogen atoms or methyl groups.

The benzotriazole compounds and benzoimidazole compounds may be used alone, or two or more thereof may be used in combination. Specific examples of the benzotriazole compounds and benzoimidazole compounds include 1H-benzotriazole (hereinafter, the term will also be referred to by the abbreviation BTA), 5-methyl-1H-benzotriazole, 5,6-dimethyl-1H-benzotriazole, benzoimidazole, 5-methylbenzoimidazole, and 5,6-dimethylbenzoimidazole. In view of the shade and storage stability of the composition, preferred are 1H-benzotriazole, and 5-methyl-1H-benzotriazole.

Examples of the sulfur-containing reducing inorganic compounds include sulfites, bisulfites, pyrosulfites, thiosulfates, thionates, and dithionites. Preferred among these are sulfites and bisulfites. Specific examples of the sulfur-containing reducing inorganic compounds include sodium sulfite, potassium suifite, calcium sulfite, ammonium sulfite, sodium bisulfite, and potassium bisulfite. The sulfur-containing reducing inorganic compounds may be used alone, or two or more thereof may be used in combination.

Examples of the thiourea compounds include ethylenethiourea, dimethylethylenethiourea, N,N'-dimethylthiourea, N,N'-diethylthiourea, N,N'-di-n-propylthiourea, dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, N-acetylthiourea, N-benzoylthiourea, diphenylthiourea, and pyridylthiourea. Preferred among these are 4,4-dimethylethylenethiourea, pyridylthiourea, and N-benzoylthiourea.

Examples of the aldehydes include terephthalaldehyde, and benzaldehyde derivatives. Examples of the benzaldehyde derivatives include dimethylaminobenzaldehyde, p-methoxybenzaldehyde, p-ethoxybenzaldehyde, and p-n-octyloxybenzaldehyde.

Examples of the thiol compounds include 3-mercaptopropyltrimethoxysilane, 2-mercaptobenzoxazole, decanethiol, and thiobenzoic acid.

Specific examples of the borate compounds, barbituric acid compounds, triazine compounds, copper compounds, tin compounds, vanadium compounds, halogen compounds, and sulfur-containing reducing inorganic compounds (for example, sulfites and bisulfites) include those mentioned in WO2008/087977.

The polymerization accelerator (f) may be contained alone, or two or more thereof may be contained in combination. The content of the polymerization accelerator (f) used in the present invention is not particularly limited. However, in view of the curability and other properties of the dental adhesive kit obtained, the content of polymerization accelerator (f) is preferably 0.001 to 30 parts by mass, more preferably 0.01 to 10 parts by mass, even more preferably 0.1 to 5 parts by mass relative to total 100 parts by mass of monomers in the self-adhesive dental composite resin (X). When the content of polymerization accelerator (f) is at or above these lower limits, it is easier to provide a sufficient bond strength by allowing polymerization to sufficiently proceed. In this respect, the content of polymerization accelerator (f) is more preferably 0.05 parts or more by mass. When the content of polymerization accelerator (f) is at or below the foregoing upper limits, it is easier to provide sufficient adhesive properties, and to reduce precipitation of the polymerization accelerator (f) itself from the self-adhesive dental composite resin (X). In this respect, the content of polymerization accelerator (f) is more preferably 20 parts or less by mass.

### <Chemical Polymerization Initiator>

A self-adhesive dental composite resin (X) of the present invention may additionally comprise a chemical polymerization initiator. The chemical polymerization initiator is not particularly limited, and may be a known chemical polymerization initiator. Examples include organic peroxides, inorganic peroxides, and transition metal complexes. Preferred are organic peroxides. The organic peroxides are not particularly limited, and may be known organic peroxides. Typical examples of organic peroxides include ketone peroxides, hydroperoxides, diacyl peroxides, dialkyl peroxides, peroxyketals, peroxyesters, and peroxydicarbonates. Specific examples of the organic peroxides, inorganic peroxides, and transition metal complexes include those exemplified in conjunction with the chemical polymerization initiator (e) of the separate-pack type dental adhesive composition (Y) described below. The chemical polymerization initiator may be used alone, or two or more thereof may be used in combination.

### <Fluorine-Ion Releasing Substance>

A self-adhesive dental composite resin (X) of the present invention may additionally comprise a fluorine-ion releasing substance. By containing a fluorine-ion releasing substance, the self-adhesive dental composite resin (X) produced can impart acid resistance to tooth structure. Examples of the fluorine-ion releasing substance include fluorine-ion releasing polymers such as a copolymer of methyl methacrylate and methacrylic acid fluoride; metal fluorides such as sodium fluoride, potassium fluoride, sodium monofluorophosphate, lithium fluoride, and ytterbium fluoride; and fluoroaluminosilicate glass. The fluorine-ion releasing substance may be contained alone, or two or more thereof may be contained in combination.

A self-adhesive dental composite resin (X) of the present invention may also comprise a known additive, provided that such additives do not cause a performance drop. Examples of additives that may be contained in the self-adhesive dental composite resin (X) include polymerization inhibitors, antioxidants, colorants (pigments, dyes), ultraviolet absorbers, solvents such as organic solvents, and thickeners. The additives may be used alone, or two or more thereof may be used in combination. In certain embodiments, the content of the solvent (for example, water, organic solvent) in the self-adhesive dental composite resin (X) is preferably less than 1 mass%, more preferably less than 0.1 mass%, even more preferably less than 0.01 mass% in total 100 mass% of the self-adhesive dental composite resin (X).

Examples of the polymerization inhibitors include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 1.0 parts by mass relative to total 100 parts by mass of monomers in the self-adhesive dental composite resin (X).

Examples of the fractions of the components of the self-adhesive dental composite resin (X) are as follows. By taking the total amount of monomers in the self-adhesive dental composite resin (X) as 100 parts by mass, it is preferable to comprise 1 to 40 parts by mass of monomer (a-1) having a phosphoric acid group, and 60 to 99 parts by mass of monomer (b) having no acidic group, alongside 0.05 to 10 parts by mass of photopolymerization initiator (c), 100 to 900 parts by mass of filler (d), and 0.001 to 30 parts by mass of polymerization accelerator (f) relative to total 100 parts by mass of the monomers. More preferably, it is preferable to comprise 2.5 to 35 parts by mass of monomer (a-1) having a phosphoric acid group, and 65 to 97.5 parts by mass of monomer (b) having no acidic group in total 100 parts by mass of the monomers, alongside 0.1 to 5 parts by mass of photopolymerization initiator (c), 120 to 560 parts by mass of filler (d), and 0.01 to 10 parts by mass of polymerization accelerator (f) relative to total 100 parts by mass of the monomers. It is even more preferable to comprise 5 to 30 parts by mass of monomer (a-1) having a phosphoric acid group, and 70 to 95 parts by mass of monomer (b) having no acidic group in total 100 parts by mass of the monomers, alongside 0.15 to 2.5 parts by mass of photopolymerization initiator (c), 150 to 400 parts by mass of filler (d), and 0.1 to 5 parts by mass of polymerization accelerator (f) relative to total 100 parts by mass of the monomers.

In view of workability, a self-adhesive dental composite resin (X) of the present invention is of a one-pack type (one-paste type) with fully pre-mixed components. In the resin coating process, a dental composite resin is typically applied to the prepared cavity surface to protect the exposed dentin and tooth pulp. When the dental composite resin used for the resin coating process is a two-pack type, it is necessary to mix the two components immediately before use in such applications. This involves the potential risk of introducing air bubbles, and may affect the properties of the cured product of the self-adhesive dental composite resin. In the present invention, the self-adhesive dental composite resin (X) is a one-pack type, making it directly applicable without the need to mix two materials. This leads to improved workability, with no risk of air bubble inclusion, and can reduce wastage during the disposal of the composite paste. Preferably, a one-pack type self-adhesive dental composite resin (X) of the present invention is filled into a cylindrical syringe container for use. The dimensions of the cylindrical part of the syringe container are preferably 10 cm in length with an inner diameter of 15 mm or less, more preferably 7.5 cm in length with an inner diameter of 10 mm or less. For improved handling, a nozzle may be attached to the tip of the syringe before use. The dimensions of the nozzle are preferably 25 mm in length with an inner diameter of 1.5 mm or less at the opening, more preferably 20 mm in length with an inner diameter of 0.75 mm or less at the opening.

### · Separate-Pack Type Dental Adhesive Composition (Y)

A separate-pack type dental adhesive composition (Y) of the present invention comprises a first agent and a second agent that are separately packed from each other, with the first agent comprising a monomer (a) having an acidic group, and a chemical polymerization initiator (e), and the second agent comprising a monomer (b) having no acidic group. By incorporating the monomer (a) having an acidic group, it is possible to have adhesive properties to tooth structure and dental prostheses.

### <Monomer (a) Having Acidic Group>

Examples of the monomer (a) having an acidic group include a monomer having at least one acidic group such as a phosphoric acid group, a pyrophosphoric acid group, a thiophosphoric acid group, a phosphonic acid group, a sulfonic acid group, and a carboxylic acid group, and at least one polymerizable group such as an acryloyl group, a methacryloyl group, a vinyl group, and a styrene group. The monomer (a) having an acidic group has an affinity for the adherend, and also exhibits a demineralizing effect on tooth structure. Specific examples of the monomer (a) having an acidic group are as follows.

Examples of monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, glycerol phosphate di(meth)acrylate, and acid chlorides, alkali metal salts, and ammonium salts of these.

Examples of monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and ammonium salts of these.

Examples of monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyeicosyl dihydrogen thiophosphate, and acid chlorides, alkali metal salts, and ammonium salts of these.

Examples of monomers having a phosphonic acid group include 2-(meth)acryloyloxyethylphenylphosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, and ammonium salts of these.

Examples of monomers having a sulfonic acid group include 2-(meth)acrylamide-2-methylpropanesulfonic acid, styrenesulfonic acid, and 2-sulfoethyl (meth)acrylate.

Examples of monomers having a carboxylic acid group include monomers having one carboxy group within the molecule, and monomers having a plurality of carboxy groups within the molecule.

Examples of monomers having one carboxy group within the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinyl benzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, 2-(meth)acryloyloxyethyl hydrogen malate, and acid halides of these.

Examples of monomers having a plurality of carboxy groups within the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1, 1-dicarboxylic acid, 12-(meth)acryloyloxydodecane-1,1-dicarboxylic acid, 13-(meth)acryloyloxytridecane-1, 1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, and acid anhydrides or acid halides of these.

The monomer (a) having an acidic group may be used alone, or two or more thereof may be used in combination. In view of high bond strength to dental adherends, the monomer (a) having an acidic group is preferably one or more selected from the group consisting of a monomer having a phosphoric acid group, a monomer having a carboxylic acid group, and a monomer having a sulfonic acid group, more preferably one or more selected from the group consisting of a monomer having a phosphoric acid group with two or more hydroxyl groups attached to the phosphorus atom, and a monomer having a plurality of carboxy groups within the molecule, even more preferably one or more selected from the group consisting of 10-(meth)acryloyloxydecyl dihydrogen phosphate, 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamide-2-methylpropanesulfonic acid, and 11-(meth)acryloyloxyundecane-1,1-dicarboxylic acid.

Preferably, the monomer (a) having an acidic group in the separate-pack type dental adhesive composition (Y) comprises a compound identical to the monomer (a-1) having a phosphoric acid group contained in the self-adhesive dental composite resin (X).

A certain preferred embodiment is, for example, a dental adhesive kit in which the monomer (a) having an acidic group in the separate-pack type dental adhesive composition (Y) comprises a monomer (a-1) having a divalent phosphoric acid group with a backbone C8 to C16 alkyl or alkylene group within the molecule.

The content of the monomer (a) having an acidic group in the separate-pack type dental adhesive composition (Y) is preferably 1 to 50 parts by mass, more preferably 2 to 30 parts by mass, even more preferably 2 to 15 parts by mass in total 100 parts by mass of monomers in a separate-pack type dental adhesive composition (Y) of the present invention. When the content of the monomer (a) having an acidic group is 1 part or more by mass, it is easier to obtain high adhesive properties to the self-adhesive dental composite resin (X). When the content of monomer (a) having an acidic group is 50 parts or less by mass, it is easier to maintain a balance between polymerizability and adhesive properties. In this specification, a total amount of monomers in separate-pack type dental adhesive composition (Y) means the total mass of the monomer (a) having an acidic group, the monomer (b) having no acidic group, and the silane coupling agent (g) (described later) contained in the first and second agents (here, the total amount of monomers includes a silane coupling agent having a polymerizable group when such a silane coupling agent, different from silane coupling agent (g), is contained in the separate-pack type dental adhesive composition (Y)).

### <Monomer (b) Having no Acidic Group>

The monomer (b) having no acidic group is a monomer that polymerizes through a radical polymerization reaction initiated by a polymerization initiator. The first agent in a separate-pack type dental adhesive composition (Y) of the present invention comprises a monomer (b) having no acidic group. A certain preferred embodiment is, for example, a dental adhesive kit in which the first and second agents of the separate-pack type dental adhesive composition (Y) comprise a monomer (b) having no acidic group. The monomer (b) having no acidic group in the separate-pack type dental adhesive composition (Y) may be used alone, or two or more thereof may be used in combination. When the first and second agents comprise a monomer (b) having no acidic group, the monomer (b) having no acidic group in the first agent may be the same or different from the monomer (b) having no acidic group in the second agent. For example, when the first and second agents each comprise two or more monomers (b) having no acidic group, the first and second agents may comprise at least one common monomer. The monomer (b) having no acidic group used for the first agent of the separate-pack type dental adhesive composition (Y) may be the same monomer (b) having no acidic group used for the self-adhesive dental composite resin (X). Preferred are hydrophobic monomer (b-2) and hydrophilic monomer (b-3). These descriptions of the monomer (b) having no acidic group contained in the first agent also apply to the monomer (b) having no acidic group contained in the second agent.

The hydrophobic monomer (b-2) improves the mechanical strength, handling, and other properties of the separate-pack type dental adhesive composition (Y).

Preferred examples of aliphatic bifunctional monomers as hydrophobic monomers (b-2) used for the separate-pack type dental adhesive composition (Y) include glycerol dimethacrylate, triethylene glycol di(meth)acrylate, neopentyl glycol dimethacrylate, 2,2,4-trimethylhexamethylenebis(2-carbamoyloxyethyl)dimethacrylate, and 1,2-bis(3-methacryloyloxy-2-hydroxypropoxy)ethane.

The hydrophilic monomer (b-3) used for the separate-pack type dental adhesive composition (Y) penetrates into tooth structure by itself, and adheres to the organic component (collagen) in the tooth structure, in addition to promoting the penetration of the components of separate-pack type dental adhesive composition (Y) into the tooth structure. Preferred as the hydrophilic monomer (b-3) of separate-pack type dental adhesive composition (Y) are hydrophilic polymerizable monofunctional (meth)acrylate monomers, more preferably 2-hydroxyethyl (meth)acrylate.

The monomer (b) having no acidic group used for the separate-pack type dental adhesive composition (Y) may be contained alone, or two or more thereof may be contained in combination. The monomer (b) having no acidic group is preferably 10 to 98 parts by mass, more preferably 50 to 95 parts by mass, even more preferably 60 to 92 parts by mass in total 100 parts by mass of monomers in a separate-pack type dental adhesive composition (Y) of the present invention. The content of hydrophobic monomer (b-2) is preferably 10 to 97 parts by mass, more preferably 50 to 95 parts by mass, even more preferably 60 to 92 parts by mass in total 100 parts by mass of monomers in the separate-pack type dental adhesive composition (Y). The content of hydrophilic monomer (b-3) is preferably 1 to 50 parts by mass, more preferably 2 to 25 parts by mass, even more preferably 3 to 10 parts by mass in total 100 parts by mass of monomers in a separate-pack type dental adhesive composition (Y) of the present invention.

When the monomer (b) having no acidic group in a separate-pack type dental adhesive composition (Y) of the present invention comprises a mixture of two or more aromatic di(meth)acrylates represented by general formula (2), it is preferable that the separate-pack type dental adhesive composition (Y) additionally comprise a silane coupling agent (g). In view of paste properties, it is preferable in such a separate-pack type dental adhesive composition (Y) that the proportion of the mixture of two or more aromatic di(meth)acrylates represented by general formula (2), and the proportion of the silane coupling agent (g) be 1:1 to 30:1, more preferably 3:1 to 25:1, even more preferably 5:1 to 23:1 in terms of a mass ratio in the total mass of the first and second agents.

The first agent in a separate-pack type dental adhesive composition (Y) of the present invention comprises a chemical polymerization initiator (e).

### <Chemical Polymerization Initiator (e)>

Examples of chemical polymerization initiator (e) include organic peroxides, inorganic peroxides, and transition metal complexes. These are not particularly limited, and may be known compounds. The chemical polymerization initiator (e) may be used alone, or two or more thereof may be used in combination.

Typical examples of organic peroxides include ketone peroxides, hydroperoxides, diacyl peroxides, dialkyl peroxides, peroxyketals, peroxyesters, and peroxydicarbonates. Particularly preferred are hydroperoxides and peroxyesters. Most preferred are peroxyesters because it allows a separate-pack type dental adhesive composition (Y) of the present invention to show little variation in operable timeframe even after extended storage. The organic peroxides may be used alone, or two or more thereof may be used in combination.

Examples of the ketone peroxides include methyl ethyl ketone peroxide, cyclohexanone peroxide, methyl cyclohexanone peroxide, methyl acetoacetate peroxide, and acetyl acetone peroxide.

Examples of the hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide (hereinafter, the term will also be referred to by the abbreviation THP).

Examples of the diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of the dialkyl peroxides include α,α-bis(t-butylperoxy)diisopropylbenzene, dicumyl peroxide, 2,5-dimethyl-2,5-bis(t-butylperoxy)hexane, t-butylcumyl peroxide, di-t-butyl peroxide, and 2,5-dimethyl-2,5-bis(t-butylperoxy)3-hexyne.

Examples of the peroxyketals include 1,1-bis(t-hexylperoxy)3,3,5-trimethylcyclohexane, 1,1-bis(t-hexylperoxy)cyclohexane, 1,1-bis(t-butylperoxy)3, 3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate, and 2,2-bis(4,4-di-t-butylperoxycyclohexyl)propane.

The peroxyesters may be any known peroxyesters, provided that the peroxy group (-OO- group) has an acyl group at one end, and a hydrocarbon group (or a similar group) at the other end. Specific examples include α,α-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexane, 1-cyclohexyl-1-methylethylperoxy-2-ethylhexanoate, t-hexyl peroxy-2-ethylhexanoate, t-butyl peroxy-2-ethylhexanoate, t-butyl peroxyisobutyrate, t-hexyl peroxyisopropyl monocarbonate, t-butyl peroxymaleic acid, t-butyl peroxy-3,5,5-trimethylhexanoate, t-butyl peroxylaurate, 2,5-dimethyl-2,5-bis(m-toluoylperoxy)hexane, t-butyl peroxyisopropyl monocarbonate, t-butyl peroxy-2-ethylhexyl monocarbonate, t-hexyl peroxybenzoate, 2,5-dimethyl-2,5-bis(benzoylperoxy)hexane, t-butyl peroxyacetate, t-butyl peroxy-m-toluoylbenzoate, t-butyl peroxybenzoate (hereinafter, the term will also be referred to by the abbreviation BPB), and bis(t-butylperoxy)isophthalate. These may be used alone, or two or more thereof may be used in appropriate combinations. In view of storage stability and reactivity, preferred are t-butyl peroxymaleic acid, t-butyl peroxy-3,5,5-trimethylhexanoate, t-butyl peroxybenzoate, t-butyl peroxyisopropyl monocarbonate, t-butyl peroxy-2-ethylhexyl monocarbonate, and t-butyl peroxyacetate, more preferably t-butyl peroxybenzoate.

Examples of the peroxydicarbonates include di-n-propylperoxydicarbonate, diisopropylperoxydicarbonate, bis(4-t-butylcyclohexyl)peroxydicarbonate, di(2-ethoxyethyl)peroxydicarbonate, di(2-ethylhexyl)peroxydicarbonate, di(2-methoxybutyl)peroxydicarbonate, and di(3-methyl-3-methoxybutyl)peroxydicarbonate.

Examples of the inorganic peroxides include peroxydisulfates and peroxydiphosphates. In view of curability, peroxydisulfates are preferred. Specific examples of peroxydisulfates include sodium peroxydisulfate, potassium peroxydisulfate (hereinafter, the term will also be referred to by the abbreviation KPS), aluminum peroxydisulfate, and ammonium peroxydisulfate.

In view of curability, the organic peroxides and inorganic peroxides are preferably 0.01 to 5 parts by mass, more preferably 0.05 to 2 parts by mass relative to total 100 parts by mass of monomers in a separate-pack type dental adhesive composition (Y) of the present invention.

Examples of the transition metal complexes include copper compounds and vanadium compounds.

The copper compounds are preferably compounds that are soluble in the monomers. Specific examples of such compounds include:
copper(II) carboxylates, for example, such as copper(II) acetate, copper(II) isobutyrate, copper(II) gluconate, copper(II) citrate, copper(II) phthalate, copper(II) tartarate, copper(II) oleate, copper(II) octylate, copper(II) octenoate, copper(II) naphthenate, copper(II) methacrylate, and copper(II) 4-cyclohexylbutyrate;
copper β-diketones, for example, such as copper(II) acetylacetonate, copper(II) trifluoroacetylacetonate, copper(II) hexafluoroacetylacetonate, copper(II) 2,2,6,6-tetramethyl-3,5-heptanedionate, and copper(II) benzoylacetonate;
copper β-ketoesters, for example, such as copper(II) ethylacetoacetate;
copper alkoxides, for example, such as copper(II) methoxide, copper(II) ethoxide, copper(II) isopropoxide, copper(II) 2-(2-butoxyethoxy)ethoxide, and copper(II) 2-(2-methoxyethoxy)ethoxide;
copper dithiocarbamates, for example, such as copper(II) dimethyldithiocarbamate;
salts of copper and inorganic acids, for example, such as copper(II) nitrate; and copper(II) chloride.

These may be used alone, or two or more thereof may be used in appropriate combinations. In view of solubility and reactivity to the monomers, preferred are copper(II) carboxylates, copper(II) β-diketones, and copper(II) β-ketoesters, and particularly preferred are copper(II) acetate and copper(II) acetylacetonate.

In view of curability, the content of the copper compound is preferably 0.000005 to 1 parts by mass relative to total 100 parts by mass of monomers (the total mass of the first agent and second agent) in a separate-pack type dental adhesive composition (Y) of the present invention.

Examples of the vanadium compounds include vanadium acetylacetonate, vanadyl acetylacetonate (hereinafter, the term will also be referred to by the abbreviation VOAA), vanadyl stearate, vanadium naphthenate, and vanadium benzoylacetonate. Particularly preferred are vanadium acetylacetonate, and vanadyl acetylacetonate.

In view of curability, the content of the vanadium compound is preferably 0.005 to 1 parts by mass relative to total 100 parts by mass of monomers (the total mass of the first agent and second agent) in a separate-pack type dental adhesive composition (Y) of the present invention.

### <Filler (d)>

Preferably, the first agent and/or second agent of a separate-pack type dental adhesive composition (Y) of the present invention comprise a filler (d).

The filler (d) used for the separate-pack type dental adhesive composition (Y) may be the same filler (d) used for the self-adhesive dental composite resin (X). The filler (d) may be used alone, or two or more thereof may be used in combination. The filler (d) of the separate-pack type dental adhesive composition (Y) may be the same or different from the filler (d) of the self-adhesive dental composite resin (X). A certain preferred embodiment is, for example, a dental adhesive kit in which the separate-pack type dental adhesive composition (Y) comprises a first agent and a second agent that are separately packed from each other, with the first agent comprising a monomer (a) having an acidic group, a monomer (b) having no acidic group, a filler (d), and a chemical polymerization initiator (e), and the second agent comprising a monomer (b) having no acidic group, and a filler (d).

Preferred as the filler (d) used for the separate-pack type dental adhesive composition (Y) are inorganic fillers, more preferably silica, or ceramics and glass containing silica as a base material.

In order to improve the curability, mechanical strength, and ease of handling, the filler (d) used for the separate-pack type dental adhesive composition (Y) may be used after a surface treatment with a known surface treatment agent. The surface treatment agent and surface treatment method may be the same surface treatment agent or surface treatment method used for the surface treatment of the inorganic fillers of the self-adhesive dental composite resin (X).

The content of the filler (d) in the separate-pack type dental adhesive composition (Y) is preferably 10 to 80 mass%, more preferably 20 to 77 mass%, even more preferably 30 to 75 mass% in total 100 mass% (the total mass of the first agent and second agent) of a separate-pack type dental adhesive composition (Y) of the present invention.

### <Polymerization Accelerator (f-1)>

The separate-pack type dental adhesive composition (Y) may comprise a polymerization accelerator (f-1) in the first agent and/or second agent. A certain embodiment is, for example, a dental adhesive kit in which the second agent of the separate-pack type dental adhesive composition (Y) comprises a polymerization accelerator (f-1). The polymerization accelerator (f-1) is not particularly limited, as long as it is a polymerization accelerator for chemical polymerization. Examples of the polymerization accelerator (f-1) include amines, sulfinic acid and salts thereof, benzotriazole compounds, benzoimidazole compounds, sulfur-containing reducing inorganic compounds, and thiourea compounds. Specific examples of amines and other such compounds include those exemplified for the polymerization accelerator (f) of the self-adhesive dental composite resin (X). The polymerization accelerator (f-1) may be used alone, or two or more thereof may be used in combination.

Examples of the amines used as the polymerization accelerator (f-1) of the separate-pack type dental adhesive composition (Y) include the same aromatic amines and aliphatic amines used for the polymerization accelerator (f) of the self-adhesive dental composite resin (X). In view of redox reactivity, preferred as aromatic amine is N, N-bis(2-hydroxyethyl)-p-toluidine.

In view of redox reactivity, preferred as aliphatic amines are tertiary aliphatic amines, particularly preferably N-methyldiethanolamine, triethanolamine, and 2-(dimethylamino)ethyl methacrylate.

The amine content is preferably 0.01 to 10 parts by mass, more preferably 0.02 to 5 parts by mass, even more preferably 0.05 to 2 parts by mass relative to total 100 parts by mass of monomers in a separate-pack type dental adhesive composition (Y) of the present invention. When the amine content is at or above these lower limits, the separate-pack type dental adhesive composition (Y) obtained can exhibit enhanced bond strength to moist bodies such as tooth structure. When the amine content is at or below the foregoing upper limits, the separate-pack type dental adhesive composition (Y) obtained can exhibit improved shade stability.

The content of sulfinic acid and salts thereof is preferably 0.1 to 5 parts by mass, more preferably 0.2 to 4 parts by mass, even more preferably 0.5 to 3 parts by mass relative to total 100 parts by mass of monomers in a separate-pack type dental adhesive composition (Y) of the present invention. When the content of sulfinic acid and salts thereof is at or above these lower limits and at or below these upper limits, the separate-pack type dental adhesive composition (Y) obtained can produce a cured product having improved mechanical strength.

The content of benzotriazole compound is preferably 0.01 to 10 parts by mass, more preferably 0.02 to 5 parts by mass, even more preferably 0.05 to 2 parts by mass relative to total 100 parts by mass of monomers in a separate-pack type dental adhesive composition (Y) of the present invention. When the content of benzotriazole compound is at or above these lower limits and at or below these upper limits, the separate-pack type dental adhesive composition (Y) obtained can produce a cured product having improved mechanical strength.

The content of benzoimidazole compound is preferably 0.01 to 10 parts by mass, more preferably 0.02 to 5 parts by mass, even more preferably 0.05 to 2 parts by mass relative to total 100 parts by mass of monomers in a separate-pack type dental adhesive composition (Y) of the present invention. When the content of benzoimidazole compound is at or above these lower limits and at or below these upper limits, the separate-pack type dental adhesive composition (Y) obtained can produce a cured product having improved mechanical strength.

The content of sulfur-containing reducing inorganic compound is preferably 0.01 to 15 parts by mass, more preferably 0.05 to 10 parts by mass, even more preferably 0.1 to 5 parts by mass relative to total 100 parts by mass of monomers in a separate-pack type dental adhesive composition (Y) of the present invention. When the content of sulfur-containing reducing inorganic compound is at or above these lower limits, the separate-pack type dental adhesive composition (Y) obtained can exhibit enhanced bond strength to moist bodies such as tooth structure. When the content of sulfur-containing reducing inorganic compound is at or below the foregoing upper limits, the separate-pack type dental adhesive composition (Y) obtained can produce a cured product having improved mechanical strength.

The content of thiourea compound is preferably 0.1 to 5 parts by mass, more preferably 0.2 to 4 parts by mass, even more preferably 0.5 to 3 parts by mass relative to total 100 parts by mass of monomers in a separate-pack type dental adhesive composition (Y) of the present invention. When the content of thiourea compound is at or above these lower limits and at or below these upper limits, the separate-pack type dental adhesive composition (Y) obtained can produce a cured product having improved mechanical strength.

### <Polymerization Accelerator (f-2) for Photopolymerization>

For improved photocurability, it is possible to use a polymerization accelerator (f-2) for photopolymerization (hereinafter, also referred to simply as "polymerization accelerator (f-2)") with a photopolymerization initiator (i) (described later). When the separate-pack type dental adhesive composition (Y) contains a photopolymerization initiator (i), the polymerization accelerator (f-2) may be contained in at least one of the first agent and second agent. For example, when the first agent contains the photopolymerization initiator (i), the second agent may contain the polymerization accelerator (f-2). The polymerization accelerator (f-2) is not particularly limited, as long as it is usable for photopolymerization. Examples include compounds that can be used for photopolymerization from among the examples of the polymerization accelerator (f) of the self-adhesive dental composite resin (X), preferably polymerization accelerators that are included in polymerization accelerator (f) but are excluded from the polymerization accelerator (f-1) of the separate-pack type dental adhesive composition (Y). Specific examples of polymerization accelerator (f-2) include aldehydes, thiol compounds, and triazine compounds (for example, triazine compounds substituted with a trihalomethyl group). The aldehydes and thiol compounds may be the same aldehydes or thiol compounds used for the polymerization accelerator (f) of the self-adhesive dental composite resin (X). The polymerization accelerator (f-2) may be used alone, or two or more thereof may be used in combination. Among the examples of triazine compounds, the triazine compound substituted with a trihalomethyl group may be any known compound, provided that it is an s-triazine compound having at least one trihalomethyl group such as a trichloromethyl group and a tribromomethyl group.

The content of triazine compound is preferably 0.005 to 0.3 parts by mass, more preferably 0.008 to 0.2 parts by mass, even more preferably 0.01 to 0.1 parts by mass relative to total 100 parts by mass of monomers in a separate-pack type dental adhesive composition (Y) of the present invention.

### <Silane Coupling Agent (g)>

Preferably, the silane coupling agent (g) is incorporated in the first or second agent of the separate-pack type dental adhesive composition (Y), in order to more greatly improve the adhesive properties of separate-pack type dental adhesive composition (Y) to coating surfaces of self-adhesive dental composite resin (X). Examples of the silane coupling agent (g) include the same silane coupling agent (g) (preferably, silane coupling agents represented by general formula (5)) used for the surface treatment of the inorganic fillers of the self-adhesive dental composite resin (X). A certain preferred embodiment is, for example, a separate-pack type dental adhesive composition (Y) in which the second agent comprises a monomer (b) having no acidic group, a filler (d), a polymerization accelerator (f-1), and a silane coupling agent (g).

In view of adhesive properties to coating surfaces of self-adhesive dental composite resin (X), particularly preferred as the silane coupling agent (g) used for separate-pack type dental adhesive composition (Y) are γ-methacryloxypropyltrimethoxysilane, γ-methacryloxypropyltriethoxysilane, 6-(meth)acryloyloxyhexyltrimethoxysilane, 6-(meth)acryloyloxyhexyltriethoxysilane, 8-(meth)acryloyloxyoctyltrimethoxysilane, 8-(meth)acryloyloxyoctyltriethoxysilane, κ-methacryloxydecyltrimethoxysilane, κ-methacryloxydecyltriethoxysilane, 11-(meth)acryloyloxyundecyltrimethoxysilane, and 11-(meth)acryloyloxyundecyltriethoxysilane, more preferably γ-methacryloxypropyltriethoxysilane, 8-(meth)acryloyloxyoctyltrimethoxysilane, 8-(meth)acryloyloxyoctyltriethoxysilane, 11-(meth)acryloyloxyundecyltrimethoxysilane, and 11-(meth)acryloyloxyundecyltriethoxysilane, even more preferably 8-(meth)acryloyloxyoctyltrimethoxysilane, 8-(meth)acryloyloxyoctyltriethoxysilane, 11-(meth)acryloyloxyundecyltrimethoxysilane, and 11-(meth)acryloyloxyundecyltriethoxysilane.

In view of providing excellent bond strength, the content of silane coupling agent (g) is preferably 1 to 50 parts by mass, more preferably 1 to 40 parts by mass, even more preferably 2 to 30 parts by mass in total 100 parts by mass of monomers in a separate-pack type dental adhesive composition (Y) of the present invention.

In view of providing excellent bond strength, the content of silane coupling agent (g) is preferably 0.1 to 10.0 mass% in total 100 mass% of a separate-pack type dental adhesive composition (Y) of the present invention. In view of the bond durability with respect to self-adhesive dental composite resin (X), the content of silane coupling agent (g) is more preferably 0.5 to 9.0 mass%, even more preferably 1.0 to 8.0 mass%, particularly preferably 1.2 to 7.0 mass%. It is to be noted here that the content of the silane coupling agent (g) incorporated in the separate-pack type dental adhesive composition (Y) excludes the silane coupling agent (g) used as a surface treatment agent for the surface treatment of the inorganic fillers of the separate-pack type dental adhesive composition (Y).

### <Photopolymerization Initiator (i)>

A photopolymerization initiator (i) may be incorporated in at least one of the first agent and second agent, in order to provide a separate-pack type dental adhesive composition (Y) of the present invention as a dual-cure composition that can also undergo polymerization by photoirradiation. Examples of the photopolymerization initiator (i) include the water-soluble photopolymerization initiator (c-1) and water-insoluble photopolymerization initiator (c-2) used for the self-adhesive dental composite resin (X), α-aminoacetophenones, and water-soluble acylphosphine oxides disclosed in JP H03-57916 B. Preferred is water-insoluble photopolymerization initiator (c-2). More preferred are α-diketones, and (bis)acylphosphine oxides other than water-soluble photopolymerization initiator (c-1).

Examples of the α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

The photopolymerization initiator (i) may be used alone, or two or more thereof may be used in combination. The content of photopolymerization initiator (i) ranges preferably from 0.005 to 10 parts by mass, more preferably from 0.01 to 5 parts by mass relative to total 100 parts by mass of monomers in a separate-pack type dental adhesive composition (Y) of the present invention.

A separate-pack type dental adhesive composition (Y) of the present invention may comprise a fluorine-ion releasing substance to provide acid resistance to tooth structure. The fluorine-ion releasing substance may be any of the fluorine-ion releasing substances exemplified for the self-adhesive dental composite resin (X).

A separate-pack type dental adhesive composition (Y) of the present invention may comprise additives such as stabilizers (polymerization inhibitors), colorants (dyes, pigments), fluorescent agents, ultraviolet absorbers, solvents (e.g., organic solvents), or thickeners. The additives may be used alone, or two or more thereof may be used in combination. The separate-pack type dental adhesive composition (Y) may also comprise anti-microbial substances such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, or triclosan. In certain embodiments, the content of the solvent (for example, water or an organic solvent) in the separate-pack type dental adhesive composition (Y) is preferably less than 1 mass%, more preferably less than 0.1 mass%, even more preferably less than 0.01 mass% in total 100 mass% of the separate-pack type dental adhesive composition (Y).

A first agent of the present invention may consist essentially of a monomer (a) having an acidic group, a monomer (b) having no acidic group, a chemical polymerization initiator (e), and a filler (d). Likewise, a second agent of the present invention may consist essentially of a monomer (b) having no acidic group, a filler (d), and a polymerization accelerator (f-1).

A separate-pack type dental adhesive composition (Y) of the present invention can be produced by, for example, mixing components other than a powdery component (e.g., filler (d)) to prepare a solution, and adding the powdery component to the solution.

A dental adhesive kit of the present invention can be suitably used for dental treatments employing the indirect restoration method using dental resin cements.

A certain preferred embodiment is, for example, a tooth restoration method that comprises the steps of:
curing a one-pack type self-adhesive dental composite resin (X);
applying a separate-pack type dental adhesive composition (Y) to a predetermined region of the cured self-adhesive dental composite resin (X); and
curing the applied separate-pack type dental adhesive composition (Y),
the self-adhesive dental composite resin (X) comprising a monomer (a-1) having a divalent phosphoric acid group with a backbone C8 to C16 alkyl or alkylene group within the molecule, a monomer (b) having no acidic group, a photopolymerization initiator (c), and a filler (d),
the separate-pack type dental adhesive composition (Y) comprising a first agent and a second agent that are separately packed from each other, with the first agent comprising a monomer (a) having an acidic group, and a chemical polymerization initiator (e), and the second agent comprising a monomer (b) having no acidic group.

The step of applying the separate-pack type dental adhesive composition (Y) is not particularly limited. For example, the separate-pack type dental adhesive composition (Y) may be applied, coated, or built up in a predetermined region of the cured self-adhesive dental composite resin (X).

The step of curing the one-pack type self-adhesive dental composite resin (X), and the step of curing the applied separate-pack type dental adhesive composition (Y) are not particularly limited, and known curing methods (e.g., photopolymerization, thermal polymerization) may be used.

The present invention encompasses embodiments combining the foregoing features in various ways within the technical idea of the present invention, provided that the present invention can exhibit its effects.

### EXAMPLES

The following describes the present invention in greater detail using Examples and Comparative Examples. However, the present invention is not limited to the following descriptions. In the following, "part(s)" means "part(s) by mass", unless otherwise specifically stated.

The components of the one-pack type self-adhesive dental composite resins (X) of Examples and Comparative Examples are presented below, along with the abbreviations.
· One-pack type self-adhesive dental composite resin (X)
   [Monomer (a-1) having a phosphoric acid group]
      MDP: 10-methacryloyloxydecyl dihydrogen phosphate
   [Monomer having an acidic group other than monomer (a-1) having a phosphoric acid group]
      GPDM: glycerol phosphate dimethacrylate
      4-META: 4-methacryloyloxyethyl trimellitate anhydride

   [Monomer (b) having no acidic group]
      Bis-GMA: 2,2-bis[4-(2-hydroxy-3-methacryloyloxypropoxy)phenyl]propane
      D-2.6E: 2,2-bis(4-methacryloyloxypolyethoxyphenyl)propane (with an average of 2.6 moles of ethoxy group added)
      3G: triethylene glycol dimethacrylate
      DD: 1,10-decanediol dimethacrylate
      MAEA: N-methacryloyloxyethylacrylamide
      DEAA: N,N-diethylacrylamide
      HEMA: 2-hydroxyethyl methacrylate
   [Photopolymerization initiator (c)]
Water-soluble photopolymerization initiator (c-1)
   · Li-TPO: Lithium phenyl(2,4,6-trimethylbenzoyl)phosphinate
· Water-insoluble photopolymerization initiator (c-2)
   CQ: dl-camphorquinone
   BAPO: bis(2,4,6-trimethylbenzoyl)phenylphosphine oxide

### [Filler (d)]

Filler 1: ultrafine particulate silica Aerosil^{®} R 972 manufactured by Nippon Aerosil Co., Ltd.; average particle diameter: 16 nm

Filler 2: silane-treated silica stone powder

A silica stone powder (silica manufactured by Nitchitsu Co., Ltd. under the trade name Hi-Silica) was pulverized with a ball mill to obtain a pulverized silica stone powder. The pulverized silica stone powder had an average particle diameter of 2.2 µm as measured by volume with a laser diffraction particle size distribution analyzer (Model SALD-2300, manufactured by Shimadzu Corporation). One-hundred parts by mass of the pulverized silica stone powder was surface treated with four parts by mass of γ-methacryloyloxypropyltrimethoxysilane by an ordinary method to obtain a filler 2.

Filler 3: A three-neck flask was charged with 100 g of GM27884 NF180 grade (a barium boroaluminosilicate glass manufactured by SCHOTT; average particle diameter: 0.18 µm), 13 g of γ-methacryloyloxypropyltrimethoxysilane, and 200 mLof a 0.3 mass% aqueous solution of acetic acid, and these were stirred at room temperature for 2 hours. After removing water by freeze drying, a heat treatment was carried out at 80°C for 5 hours to obtain a filler 3 (average particle diameter: 0.18 µm).

Filler 4: A three-neck flask was charged with 100 g of 8235 UF0.7 grade (barium boroaluminosilicate glass manufactured by SCHOTT; average particle diameter: 0.7 µm), 6 g of γ-methacryloyloxypropyltrimethoxysilane, and 200 mL of a 0.3 mass% aqueous solution of acetic acid, and these were stirred at room temperature for 2 hours. After removing water by freeze drying, a heat treatment was carried out at 80°C for 5 hours to obtain a filler 4 (average particle diameter: 0.7 µm).

### [Polymerization accelerator (f)]

DABE: ethyl 4-(N,N-dimethylamino)benzoate

### [Polymerization inhibitor]

BHT: 3,5-di-t-butyl-4-hydroxytoluene

The components of the separate-pack type dental adhesive compositions (Y) of Examples and Comparative Examples are presented below, along with the abbreviations.
· Separate-pack type dental adhesive composition (Y)
   [Monomer (a) having an acidic group]
   MDP: 10-methacryloyloxydecyl dihydrogen phosphate
   GPDM: glycerol phosphate dimethacrylate
[Monomer (b) having no acidic group]
   HEMA: 2-hydroxyethyl methacrylate
   Bis-GMA: 2,2-bis[4-(2-hydroxy-3-methacryloyloxypropoxy)phenyl]propane
   D-2.6E: 2,2-bis(4-methacryloyloxypolyethoxyphenyl)propane (with an average of 2.6 moles of ethoxy group added)
   NPG: neopentyl glycol dimethacrylate

### [Filler (d)]

Filler 5: A quartz (manufactured by MARUWA QUARTZ) was pulverized with a ball mill to obtain a quartz powder measuring about 4.5 µm in average particle diameter. One-hundred parts by mass of this quartz powder was surface treated with three parts by mass of γ-methacyloyloxypropyltrimethoxysilane by an ordinary method to obtain a filler 5.

Filler 6: A bariumsilicate glass (manufactured by Esstech, product code Raysorb E-3000) was pulverized with a ball mill to obtain a barium glass powder measuring about 2.4 µm in average particle diameter. One-hundred parts by mass of this barium glass powder was surface treated with three parts by mass of γ-methacryloyloxypropyltrimethoxysilane by an ordinary method to obtain a filler 6.

Filler 7: aluminum oxide, manufactured by Nippon Aerosil Co., Ltd. under the trade name AEROXIDE Alu C; average particle diameter: 13 nm
[Chemical polymerization initiator (e)]
   copper(II) acetate
   VOAA: vanadylacetylacetonate
   BPB: t-butyl peroxybenzoate
   BPO: benzoyl peroxide
   KPS: potassium peroxydisulfate
   THP: 1,1,3,3-tetramethylbutyl hydroperoxide
[Polymerization accelerator (f-1)]
   TPBSS: sodium 2,4,6-triisopropylbenzenesulfinate
   DEPT: N,N-bis(2-hydroxyethyl)-p-toluidine
   BTA: 1H-benzotriazole
   DMETU: 4,4-dimethylethylenethiourea
[Silane coupling agent (g)]
   γ-MPS: γ-methacyloyloxypropyltrimethoxysilane
   8-MOS: 8-methacryloyloxyoctyltrimethoxysilane
   11-MUS: 11-methacryloyloxyundecyltrimethoxysilane
[Photopolymerization initiator (i)]
   CQ: dl-camphorquinone

### [Other]

DABE: ethyl 4-(N,N-dimethylamino)benzoate (polymerization accelerator for photopolymerization)

BHT: 3,5-di-t-butyl-4-hydroxytoluene (stabilizer)

### [Examples 1 to 22 and Comparative Examples 1 to 7]

### [Preparation of separate-pack type dental adhesive composition (Y)]

The first and second agents of the compositions shown in Table 1 were prepared. For the preparation of the first agent, components other than the powdery component (filler) were formulated, and stirred to obtain a homogenous solution. The powdery component was then kneaded into the mixture, followed by defoaming. The powdery component in the first agent occurred in the form of a powder by being dispersed. For the preparation of the second agent, components other than the powdery components (filler and TPBSS) were formulated, and stirred to obtain a homogenous solution. The powdery components were then kneaded into the mixture, followed by defoaming. The powdery components in the second agent occurred in the form of a powder by being dispersed. The two agents were separately filled into a double syringe (a 5 mL double syringe manufactured by SULZER MIXPAC). After setting the plunger, a mixing tip (manufactured by SULZER MIXPAC) was attached to the tip of the double syringe, and the two agents were automatically mixed in a 1:1 mass ratio. The resulting mixture was used as a dental adhesive composition to examine its properties, following the methods of Test Example 1 below. The results are presented in Table 1.

### [Preparation of one-pack type self-adhesive dental composite resin (X)]

The raw materials shown in Tables 2 to 4 were mixed and kneaded in the dark at ordinary temperature (23°C) to prepare a one-pack type self-adhesive dental composite resin in paste form, and its properties were examined following the methods of Test Examples 1 and 2 below. The results are presented in Tables 2 to 4.

### Test Example 1: Flexural Modulus

### 1-1) Light Curing

The flexural modulus was evaluated by conducting a flexure test in compliance with ISO 4049:2009, specifically as follows. The prepared paste (a composition for one-pack type self-adhesive dental composite resin) was filled into a SUS die (2 mm in length × 25 mm in width × 2 mm in thickness), and glass slides were pressed against the paste from the top and bottom (over a 2 mm × 25 mm surface). The paste was cured by applying light through the glass slides from both sides, 5 points on each side, 10 seconds each, using a dental visible-light irradiator PenCure 2000 (manufactured by J. Morita Corp.). The resultant cured product was then measured for flexural modulus by conducting a three-point flexural test at a span length of 20 mm and a crosshead speed of 1 mm/min, using a universal testing machine (Autograph AG-I, 100kN, manufactured by Shimadzu Corporation) (n = 5), and a mean value was calculated.

### 1-2) Chemical Curing

The flexural modulus was evaluated by a flexure test in compliance with ISO 4049:2009, specifically as follows. A dental adhesive composition (separate-pack type dental adhesive composition) obtained by automatically mixing the prepared pastes of first and second agents in a 1:1 mass ratio with a mixing tip was filled into a SUS die (2 mm in length × 25 mm in width × 2 mm in thickness). Thereafter, glass slides were pressed against the paste from the top and bottom (over a 2 mm × 25 mm surface), and were secured with a 25 mm-wide binder clip. The sample, secured with the binder clip, was left to stand in a 37°C thermostatic chamber for 1 hour to polymerize and cure. After taking the sample out of the thermostatic chamber, the polymerized and cured composition was removed from the die, and was stored by keeping it immersed in 37°C distilled water for 24 hours. The resultant cured product was then measured for flexural modulus by conducting a three-point flexural test at a span length of 20 mm and a crosshead speed of 1 mm/min, using a universal testing machine (Autograph AG-I, 100kN, manufactured by Shimadzu Corporation) (n = 5), and a mean value was calculated.

### Test Example 2: Water sorption

The strength was evaluated by a water sorption test in compliance with ISO4049:2009. The one-pack type self-adhesive dental composite resin was placed inside a metal die (15 mm in diameter, 1 mm in thickness) over a polyester film on a glass slide. After laying another polyester film over the resin, another glass slide was placed, and pressed against the resin to remove the excess paste. The sample was then cured by applying light from both sides, 9 points on each side, 10 seconds each, using the irradiator PenCure 2000. Immediately after exposure, the sample was placed in a thermostatic chamber (37 ± 2°C) without removing it from the die. After 15 minutes from the start of light exposure, the sample was removed from the die to obtain a cured one-pack type self-adhesive dental composite resin.

The cured product was kept in a 37°C desiccator until it had a stable mass, and this mass was measured (mass A). Subsequently, the cured product was immersed in 37°C water for 7 days. Following immersion, the cured product was dried until it reached a stable mass, and its mass was measured (mass B), in the same way as before immersion. The water sorption of the cured product was measured using the following formula. Water sorption of cured product (µg/mm3) = {(mass B - mass A) ÷ cured product volume}

### Test Example 3: Tensile Bond Strength of Dental Resin Cement to Coating Surface of Self-Adhesive Dental Composite Resin

A hydroxyapatite plate (a HAp plate, apatite pellet APP-610, 13 mm in diameter × 2 mm, manufactured by HOYATechnosurgical Corporation) was polished with silicon carbide paper #1000 (manufactured by Nihon Kenshi Co., Ltd.) under running water. To provide a smooth surface, the polished surface was dried by blowing air and removing water from the surface. After drying, a 0.5 mm-thick Viton rubber (fluororubber) having a round hole with a 7 mm diameter was placed on the smooth surface, and the self-adhesive dental composite resin prepared in each Example or Comparative Example was filled into the hole. After placing a release film (polyester) over the resin, a glass slide was placed on the release film, and pressed against the resin to flatten the applied surface of the self-adhesive dental composite resin. The self-adhesive dental composite resin was then cured into a cured product by applying light for 10 seconds through the release film, using a dental LED photoirradiator (manufactured by J. Morita Corp. under the trade name PenCure 2000).

A bonding area was defined by attaching an adhesive tape, approximately 150 µm thick and having a round hole with a 3 mm diameter, to the surface of the cured self-adhesive dental composite resin (hereinafter, also referred to as "resin coating surface"). A cylindrical stainless steel rod (measuring 7 mm in diameter and 2.5 cm in length) was then bonded at its one end face (circular cross section) to the bonding surface, using the dental resin cement (separate-pack type dental adhesive composition (Y)) prepared in each Example or Comparative Example. After bonding, the sample was left to stand at room temperature for 30 minutes, and was immersed in distilled water to obtain a sample for adhesion testing. Here, a total of ten adhesion test samples were prepared. For the evaluation of bond durability, the tensile bond strength was measured after the samples were left to stand in a thermostatic chamber held at 37°C, and subjected to 4,000 cycles of a thermal process by alternately immersing the samples for 1 minute in 4°C cold water and in 60°C hot water.

In the measurement of tensile bond strength using the adhesion test samples, the tensile bond strength was determined as a mean value of tensile bond strengths measured with a universal testing machine (Autograph AG-I 100kN, Shimadzu Corporation) with the crosshead speed set to 2 mm/min. When the interfacial failure rate yielded a value other than 0% in the assessment of the interfacial failure rate of HAp plate-self-adhesive dental composite resin described below, it was assumed that the interface between the HAp plate and the self-adhesive dental composite resin failed before the tensile bond strength of the dental resin cement to the resin coating surface could be measured. These instances were deemed "unmeasurable".

The preferred tensile bond strength of the dental resin cement to the resin coating surface is 20 MPa or more, more preferably 25 MPa or more, even more preferably 30 MPa or more.

Test Example 4: Interfacial Failure Rate of HAp Plate-Self-Adhesive Dental Composite Resin

For each of the adhesion test samples measured for tensile bond strength in relation to bond durability, the state of the interface between the HAp plate and the self-adhesive dental composite resin (X) was visually inspected after the test, and the failure rate (%) at the interface was calculated using the following formula.

Interfacial failure rate (%) of HAp plate-self-adhesive dental composite resin (X) = {(Number of samples with failure at HAp plate-self-adhesive dental composite resin (X) interface)/10} × 100

An interfacial failure rate of 0% at the HAp plate-self-adhesive dental composite resin (X) interface means that the self-adhesive dental composite resin (X) is showing good adhesive properties to HAp.

**[Table 1]**

| Components (parts by mass) | | | Composition 1 | Composition 2 | Composition 3 | Composition 4 | Composition 5 | Composition 6 | Composition 7 | Composition 8 | Composition 9 |
|---|---|---|---|---|---|---|---|---|---|---|---|
| First agent | Monomer (a) having acidic group | MDP | 20 | 20 | 20 | 20 | 20 | 20 | 20 | - | 20 |
| | | GPDM | - | - | - | - | - | - | - | 20 | - |
| | Monomer (b) having no acidic group | Bis-GMA | 40 | 40 | 40 | 40 | 40 | 40 | 40 | 40 | 40 |
| | | D-2.6E | 25 | 25 | 25 | 25 | 25 | 25 | 25 | 25 | 25 |
| | | HEMA | 15 | 15 | 15 | 15 | 15 | 15 | 15 | 15 | 15 |
| | Filler (d) | Filler 1 | 15 | 15 | 15 | 15 | 15 | 15 | 15 | 15 | 15 |
| | | Filler 5 | 215 | 215 | 215 | 215 | 215 | 215 | 215 | 215 | 255 |
| | Chemical polymerization initiator (e) | Copper(II) acetate | 0.001 | - | - | - | 0.001 | 0.001 | 0.001 | 0.001 | 0.001 |
| | | VOAA | - | - | - | 0.1 | - | - | - | - | - |
| | | BPB | 0.5 | - | - | - | 0.5 | 0.5 | 0.5 | 0.5 | 1.5 |
| | | BPO | - | 3 | - | - | - | - | - | - | - |
| | | KPS | - | - | 2 | - | - | - | - | - | - |
| | | THP | - | - | - | 4 | - | - | - | - | - |
| | Photopolymerization initiator (i) | CQ | 0.1 | 0.1 | 0.1 | 0.1 | 0.1 | 0.1 | 0.1 | 0.1 | 0.1 |
| | Other | BHT | 0.15 | 0.15 | 0.15 | 0.15 | 0.15 | 0.15 | 0.15 | 0.15 | 0.15 |
| Second agent | Monomer (b) having no acidic group | D-2.6E | 75 | 75 | 75 | 75 | 80 | 80 | 80 | 75 | 75 |
| | | NPG | 25 | 25 | 25 | 25 | - | - | - | 25 | 25 |
| | Filler (d) | Filler 6 | 220 | 220 | 220 | 220 | 220 | 220 | 220 | 220 | 250 |
| | | Filler 7 | 10 | 10 | 10 | 10 | 10 | 10 | 10 | 10 | 10 |
| | Polymerization accelerator (f-1) | TPBSS | 3 | 3 | 3 | - | 3 | 3 | 3 | 3 | 3 |
| | | DEPT | 0.5 | 0.5 | 0.5 | - | 0.5 | 0.5 | 0.5 | 0.5 | 0.5 |
| | | BTA | 3 | - | - | - | 3 | 3 | 3 | 3 | 3 |
| | | DMETU | - | - | - | 4 | - | - | - | - | - |
| | Silane coupling agent (g) | γ-MPS | - | - | - | - | 20 | - | - | - | - |
| | | 8-MOS | - | - | - | - | - | 20 | - | - | - |
| | | 11-MUS | - | - | - | - | - | - | 20 | - | - |
| | Other | DABE | 0.05 | 0.05 | 0.05 | 0.05 | 0.05 | 0.05 | 0.05 | 0.05 | 0.05 |
| | | BHT | 0.05 | 0.05 | 0.05 | 0.05 | 0.05 | 0.05 | 0.05 | 0.05 | 0.05 |
| Flexural modulus | | GPa | 5.2 | 5.6 | 4.7 | 5.5 | 4.8 | 4.3 | 3.8 | 5.7 | 6.7 |

**[Table 2]**

| Components (parts by mass) | | | Example 1 | Example 2 | Example 3 | Example 4 | Example 5 | Example 6 |
|---|---|---|---|---|---|---|---|---|
| Separate-pack type dental adhesive composition (Y) | | | Composition 1 | Composition 1 | Composition 1 | Composition 1 | Composition 1 | Composition 1 |
| Self-adhesive dental composite resin (X) | Monomer (a-1) having phosphoric acid group | MDP | 10 | 10 | 10 | 10 | 10 | 10 |
| | Monomer (b) having no acidic group | Bis-GMA | - | - | - | - | - | 30 |
| | | D-2.6E | 30 | 30 | 30 | 30 | 30 | - |
| | | 3G | 20 | 20 | 20 | 20 | 50 | 20 |
| | | MAEA | 10 | 10 | 10 | 10 | 10 | 10 |
| | | DD | 30 | 30 | 30 | 30 | - | 30 |
| | | HEMA | - | - | - | - | - | - |
| | | DEAA | - | - | - | - | - | - |
| | Water-soluble photopolymerization initiator (c-1) | Li-TPO | - | - | - | - | - | - |
| | Water-insoluble photopolymerization initiator (c-2) | CQ | 0.2 | 0.2 | 0.2 | 0.2 | 0.2 | 0.2 |
| | | BAPO | - | - | - | - | - | - |
| | Filler (d) | Filler 1 | 10 | 10 | 10 | - | 40 | 10 |
| | | Filler 2 | 250 | - | - | - | 220 | 250 |
| | | Filler 3 | - | 180 | - | 90 | - | - |
| | | Filler 4 | - | - | 200 | 100 | - | - |
| | Polymerization accelerator (f) | DABE | 0.4 | 0.4 | 0.4 | 0.4 | 0.4 | 0.4 |
| | Other | BHT | 0.05 | 0.05 | 0.05 | 0.05 | 0.05 | 0.05 |
| Flexural modulus of cured self-adhesive dental composite resin (X) | | GPa | 5.6 | 5.2 | 5.4 | 5.4 | 5.8 | 5.2 |
| Water sorption of cured self-adhesive dental composite resin (X) | | µg/mm³ | 18 | 26 | 24 | 20 | 29 | 19 |
| Tensile bond strength of dental resin cement to resin coating surface | | MPa | 31 | 27 | 29 | 28 | 30 | 29 |
| Interfacial failure rate of HAp plate-self-adhesive dental composite resin | | % | 0 | 0 | 0 | 0 | 0 | 0 |

**[Table 2] Continued**

| Components (parts by mass) | | | Example 7 | Example 8 | Example 9 | Example 10 | Example 11 |
|---|---|---|---|---|---|---|---|
| Separate-pack type dental adhesive composition (Y) | | | Composition 1 | Composition 1 | Composition 1 | Composition 1 | Composition 1 |
| Self-adhesive dental composite resin (X) | Monomer (a-1) having phosphoric acid group | MDP | 10 | 30 | 10 | 10 | 10 |
| | Monomer (b) having no acidic group | Bis-GMA | - | - | - | - | - |
| | | D-2.6E | 30 | 30 | 30 | 30 | 30 |
| | | 3G | - | 30 | 20 | 20 | 15 |
| | | MAEA | 10 | 10 | 10 | 10 | 10 |
| | | DD | 50 | - | 20 | 20 | - |
| | | HEMA | - | - | 10 | - | 25 |
| | | DEAA | - | - | - | 10 | - |
| | Water-soluble photopolymerization initiator (c-1) | Li-TPO | - | - | - | - | - |
| | Water-insoluble photopolymerization initiator (c-2) | CQ | 0.2 | 0.2 | 0.2 | 0.2 | 0.2 |
| | | BAPO | - | - | - | - | - |
| | Filler (d) | Filler 1 | 10 | 10 | 10 | 10 | 10 |
| | | Filler 2 | 250 | 250 | 250 | 250 | 250 |
| | | Filler 3 | - | - | - | - | - |
| | | Filler 4 | - | - | - | - | - |
| | Polymerization accelerator (f) | DABE | 0.4 | 0.4 | 0.4 | 0.4 | 0.4 |
| | Other | BHT | 0.05 | 0.05 | 0.05 | 0.05 | 0.05 |
| Flexural modulus of cured self-adhesive dental composite resin (X) | | GPa | 5.0 | 4.5 | 5.2 | 5.2 | 4.3 |
| Water sorption of cured self-adhesive dental composite resin (X) | | µg/mm³ | 17 | 29 | 29 | 27 | 38 |
| Tensile bond strength of dental resin cement to resin coating surface | | MPa | 28 | 27 | 26 | 27 | 26 |
| Interfacial failure rate of HAp plate-self-adhesive dental composite resin | | % | 0 | 0 | 0 | 0 | 0 |

**[Table 3]**

| Components (parts by mass) | | | Example 12 | Example 13 | Example 14 | Example 15 | Example 16 | Example 17 |
|---|---|---|---|---|---|---|---|---|
| Separate-pack type dental adhesive composition (Y) | | | Composition 1 | Composition 1 | Composition 1 | Composition 1 | Composition 2 | Composition 3 |
| Self-adhesive dental composite resin (X) | Monomer (a-1) having phosphoric acid group | MDP | 10 | 10 | 10 | 10 | 10 | 10 |
| | Monomer (b) having no acidic group | Bis-GMA | - | - | - | - | - | - |
| | | D-2.6E | - | - | 30 | 30 | 30 | 30 |
| | | 3G | - | - | 20 | 20 | 50 | 50 |
| | | MAEA | 10 | 10 | - | 10 | 10 | 10 |
| | | DD | 40 | 40 | 40 | 30 | - | - |
| | | HEMA | 40 | 40 | - | - | - | - |
| | | DEAA | - | - | - | - | - | - |
| | Water-soluble photopolymerization initiator (c-1) | Li-TPO | - | - | - | 0.2 | - | - |
| | Water-insoluble photopolymerization initiator (c-2) | CQ | 0.2 | 0.2 | 0.2 | 0.2 | 0.2 | 0.2 |
| | | BAPO | - | - | - | - | - | - |
| | Filler (d) | Filler 1 | 10 | 10 | 10 | 10 | 10 | 10 |
| | | Filler 2 | 250 | 250 | 250 | 250 | - | - |
| | | Filler 3 | - | - | - | - | 180 | 180 |
| | | Filler 4 | - | - | - | - | - | - |
| | Polymerization accelerator (f) | DABE | 0.4 | 0.2 | 0.4 | 0.4 | 0.4 | 0.4 |
| | Other | BHT | 0.05 | 0.05 | 0.05 | 0.05 | 0.05 | 0.05 |
| Flexural modulus of cured self-adhesive dental composite resin (X) | | GPa | 3.5 | 2.6 | 4.8 | 5.7 | 5.2 | 5.2 |
| Water sorption of cured self-adhesive dental composite resin (X) | | µg/mm³ | 16 | 20 | 18 | 18 | 18 | 18 |
| Tensile bond strength of dental resin cement to resin coating surface | | MPa | 26 | 25 | 26 | 32 | 29 | 28 |
| Interfacial failure rate of HAp plate-self-adhesive dental composite resin | | % | 0 | 0 | 0 | 0 | 0 | 0 |

**[Table 3] Continued**

| Components (parts by mass) | | | Example 18 | Example 19 | Example 20 | Example 21 | Example 22 |
|---|---|---|---|---|---|---|---|
| Separate-pack type dental adhesive composition (Y) | | | Composition 4 | Composition 5 | Composition 6 | Composition 7 | Composition 8 |
| Self-adhesive dental composite resin (X) | Monomer (a-1) having phosphoric acid group | MDP | 10 | 10 | 10 | 10 | 10 |
| | Monomer (b) having no acidic group | Bis-GMA | - | - | - | - | - |
| | | D-2.6E | 30 | 30 | 30 | 30 | 30 |
| | | 3G | 50 | 50 | 50 | 50 | 50 |
| | | MAEA | 10 | 10 | 10 | 10 | 10 |
| | | DD | - | - | - | - | - |
| | | HEMA | - | - | - | - | - |
| | | DEAA | - | - | - | - | - |
| | Water-soluble photopolymerization initiator (c-1) | Li-TPO | - | - | - | - | - |
| | Water-insoluble photopolymerization initiator (c-2) | CQ | 0.2 | 0.2 | 0.2 | 0.2 | 0.2 |
| | | BAPO | - | - | - | - | - |
| | Filler (d) | Filler 1 | 10 | 10 | 10 | 10 | 10 |
| | | Filler 2 | - | - | - | - | - |
| | | Filler 3 | 180 | 180 | 180 | 180 | 180 |
| | | Filler 4 | - | - | - | - | - |
| | Polymerization accelerator (f) | DABE | 0.4 | 0.4 | 0.4 | 0.4 | 0.4 |
| | Other | BHT | 0.05 | 0.05 | 0.05 | 0.05 | 0.05 |
| Flexural modulus of cured self-adhesive dental composite resin (X) | | GPa | 5.2 | 5.2 | 5.2 | 5.2 | 5.2 |
| Water sorption of cured self-adhesive dental composite resin (X) | | µg/mm³ | 18 | 18 | 18 | 18 | 18 |
| Tensile bond strength of dental resin cement to resin coating surface | | MPa | 28 | 35 | 36 | 36 | 21 |
| Interfacial failure rate of HAp plate-self-adhesive dental composite resin | | % | 0 | 0 | 0 | 0 | 0 |

**[Table 4]**

| Components (parts by mass) | | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|
| Separate-pack type dental adhesive composition (Y) | | | Composition 1 | Composition 1 | Composition 1 | Composition 1 | Composition 2 | Composition 1 | Composition 9 |
| Self-adhesive dental composite resin (X) | Monomer (a-1) having phosphoric acid group | MDP | 10 | 10 | - | - | - | 10 | 10 |
| | Monomer (a) having acidic group | GPDM | - | - | 10 | - | 10 | - | - |
| | | 4-META | - | - | - | 10 | - | - | - |
| | Monomer (b) having no acidic group | Bis-GMA | 30 | 40 | - | - | - | 30 | - |
| | | D-2.6E | - | - | 30 | 30 | 30 | - | 30 |
| | | 3G | 50 | 50 | 50 | 50 | 50 | 30 | 50 |
| | | MAEA | 10 | - | 10 | 10 | 10 | - | 10 |
| | | DD | - | - | - | - | - | - | - |
| | | HEMA | - | - | - | - | - | 40 | - |
| | | DEAA | - | - | - | - | - | - | - |
| | Photopolymerization initiator (c) | CQ | 0.2 | 0.2 | 0.2 | 0.2 | 0.2 | 0.2 | 0.2 |
| | | BAPO | - | - | - | - | - | - | - |
| | Filler (d) | Filler 1 | 10 | 10 | 10 | 10 | 10 | 10 | 10 |
| | | Filler 2 | 250 | - | - | - | - | 250 | - |
| | | Filler 3 | - | 180 | 180 | 180 | 180 | - | 180 |
| | | Filler 4 | - | - | - | - | - | - | - |
| | Polymerization accelerator (f) | DABE | 0.4 | 0.4 | 0.4 | 0.4 | 0.4 | 0.4 | 0.4 |
| | Other | BHT | 0.05 | 0.05 | 0.05 | 0.05 | 0.05 | 0.05 | 0.05 |
| Flexural modulus of cured self-adhesive dental composite resin (X) | | GPa | 8.2 | 7.8 | 8.7 | 8.5 | 8.7 | 6.6 | 5.6 |
| Water sorption of cured self-adhesive dental composite resin (X) | | µg/mm³ | 42 | 38 | 37 | 36 | 37 | 56 | 18 |
| Tensile bond strength of dental resin cement to resin coating surface | | MPa | 16 | 15 | Unmeasurable | Unmeasurable | Unmeasurable | 14 | 16 |
| Interfacial failure rate of HAp plate-self-adhesive dental composite resin | | % | 0 | 0 | 100 | 100 | 100 | 0 | 0 |

As can be seen from the results shown in Tables 2 and 3, the one-pack type self-adhesive dental composite resins (X) of Examples had a flexural modulus of 2.6 to 5.8 GPa, and the water sorption was 38 µg/mm³ or less. The separate-pack type dental adhesive compositions (Y) had a flexural modulus of 3.8 to 5.7 GPa, and the tensile bond strength of the dental resin cement to the coating surface of the self-adhesive dental composite resin was 21 MPa or higher, demonstrating superior bond durability. In Examples, the interfacial failure rate of HAp plate-self-adhesive dental composite resin was 0%, confirming that the self-adhesive dental composite resin (X) exhibits good adhesive properties to tooth structure. In contrast to Examples, the dental resin cement was shown to have a low tensile bond strength of 16 MPa or less with respect to the resin coating surface in Comparative Examples 1 and 2, in which the flexural modulus of the one-pack type self-adhesive dental composite resin was 7.8 GPa or higher, as shown in Table 4. The dental resin cement also yielded a low tensile bond strength of 16 MPa or less with respect to the resin coating surface in Comparative Example 6, in which the one-pack type self-adhesive dental composite resin had a flexural modulus of 6.6 GPa, and in Comparative Example 7, in which the separate-pack type dental adhesive composition (Y) had a flexural modulus of 6.7 GPa (composition 9). In Comparative Examples 3 to 5 in which the monomer (a-1) having a phosphoric acid group was absent, the interfacial failure rate of HAp plate-self-adhesive dental composite resin was 100%, and the tensile bond strength of the dental resin cement to the resin coating surface was unmeasurable.

### INDUSTRIAL APPLICABILITY

A dental adhesive kit of the present invention can be suitably used for the indirect restoration method in dental treatments.

## Claims

1. A dental adhesive kit comprising:
a one-pack type self-adhesive dental composite resin (X) that comprises a monomer (a-1) having a divalent phosphoric acid group with a backbone C8 to C16 alkyl or alkylene group within the molecule, a monomer (b) having no acidic group, a photopolymerization initiator (c), and a filler (d); and
a separate-pack type dental adhesive composition (Y) that comprises a first agent and a second agent that are separately packed from each other, with the first agent comprising a monomer (a) having an acidic group, and a chemical polymerization initiator (e), and the second agent comprising a monomer (b) having no acidic group,
wherein a cured product of the self-adhesive dental composite resin (X) after light curing has a flexural modulus ranging from 1.5 to 6 GPa, and
a cured product of the separate-pack type dental adhesive composition (Y) after chemical curing has a flexural modulus ranging from 1.5 to 6 GPa.

2. The dental adhesive kit according to claim 1, wherein a cured product of the separate-pack type dental adhesive composition (Y) after chemical curing has a flexural modulus ranging from 3 to 6 GPa.

3. The dental adhesive kit according to claim 1 or 2, wherein the self-adhesive dental composite resin (X) and the separate-pack type dental adhesive composition (Y) have a flexural modulus ratio ((X)/(Y)) of 0.5 to 3.0 as a ratio of a flexural modulus of a cured product of the self-adhesive dental composite resin (X) and a flexural modulus of a cured product of the separate-pack type dental adhesive composition (Y).

4. The dental adhesive kit according to any one of claims 1 to 3, wherein the content of the filler (d) is 50 mass% or more in total 100 mass% of the self-adhesive dental composite resin (X).

5. The dental adhesive kit according to any one of claims 1 to 4, wherein a cured product of the self-adhesive dental composite resin (X) has a water sorption of 50 µg/mm³ or less.

6. The dental adhesive kit according to any one of claims 1 to 5, wherein the monomer (a) having an acidic group contained in the separate-pack type dental adhesive composition (Y) comprises at least one selected from the group consisting of a monomer having a phosphoric acid group, a monomer having a carboxylic acid group, and a monomer having a sulfonic acid group.

7. The dental adhesive kit according to claim 6, wherein the monomer (a) having an acidic group contained in the separate-pack type dental adhesive composition (Y) comprises a compound identical to the monomer (a-1) having a phosphoric acid group contained in the self-adhesive dental composite resin (X).

8. The dental adhesive kit according to any one of claims 1 to 7, wherein the chemical polymerization initiator (e) comprises an organic peroxide and/or an inorganic peroxide.

9. The dental adhesive kit according to any one of claims 1 to 8, wherein the first agent of the separate-pack type dental adhesive composition (Y) additionally comprises a filler (d).

10. The dental adhesive kit according to any one of claims 1 to 9, wherein the first agent of the separate-pack type dental adhesive composition (Y) additionally comprises a monomer (b) having no acidic group.

11. The dental adhesive kit according to any one of claims 1 to 10, wherein the first or second agent of the separate-pack type dental adhesive composition (Y) additionally comprises a silane coupling agent (g).

12. A tooth restoration method comprising the steps of:
curing a one-pack type self-adhesive dental composite resin (X);
applying a separate-pack type dental adhesive composition (Y) to a predetermined region of the cured self-adhesive dental composite resin (X); and
curing the applied separate-pack type dental adhesive composition (Y),
the self-adhesive dental composite resin (X) comprising a monomer (a-1) having a divalent phosphoric acid group with a backbone C8 to C16 alkyl or alkylene group within the molecule, a monomer (b) having no acidic group, a photopolymerization initiator (c), and a filler (d),
the separate-pack type dental adhesive composition (Y) comprising a first agent and a second agent that are separately packed from each other, with the first agent comprising a monomer (a) having an acidic group, and a chemical polymerization initiator (e), and the second agent comprising a monomer (b) having no acidic group.
